(19) Europäisches Patentamt
European Patent Office
Office européen des brevets

(11) Veröffentlichungsnummer: **0 282 897 A2**

(12) # EUROPÄISCHE PATENTANMELDUNG

(21) Anmeldenummer: **88103735.2**

(22) Anmeldetag: **09.03.88**

(51) Int. Cl.⁴: **C07C 59/84** , C07C 59/88 , C07C 69/738

(30) Priorität: **20.03.87 CH 1071/87**

(43) Veröffentlichungstag der Anmeldung: **21.09.88 Patentblatt 88/38**

(84) Benannte Vertragsstaaten: **AT BE CH DE ES FR GB GR IT LI LU NL SE**

(71) Anmelder: **F. HOFFMANN-LA ROCHE & CO. Aktiengesellschaft**

**CH-4002 Basel(CH)**

(72) Erfinder: **Fischli, Albert, Prof. Dr.**
**Im Wenkenberg 20**
**CH-4125 Riehen(CH)**
Erfinder: **Gutknecht, Eva-Maria, Dr.**
**Im Feldele 23**
**D-7845 Buggingen-Seefelden(DE)**
Erfinder: **Obrecht, Daniel, Dr.**
**Frohburgstrasse 45**
**CH-4052 Basel(CH)**

(74) Vertreter: **Lederer, Franz, Dr. et al**
**Patentanwält Dr. Franz Lederer Lucile**
**Grahnstrasse 22**
**D-8000 München 80(DE)**

(54) **Aethinyl- und Aethenyl-Styryl-ketone.**

(57) Styrylketone der Formel

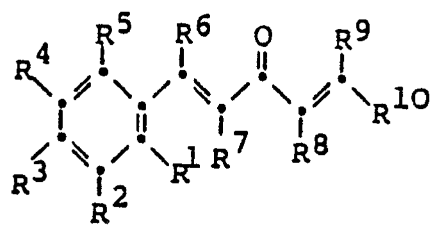

I

worin
$R^8$ und $R^9$ je Wasserstoff oder niederes Alkyl oder zusammen eine zusätzliche Kohlenstoff-Kohlenstoff-Bindung bedeuten und
$R^{10}$ einen Rest der Formel

**EP 0 282 897 A2**

$-COOR^{11}$, $-CONR^{12}R^{13}$, $-C(R^{14})=O$,

(a) (b) (c)

$-C(R^{15})(OR^{16})_2$, $-C(OR^{17})_3$ oder

(d) (e)

$-C(R^{18})(R^{19})OR^{20}$

(f)

bedeutet;
sowie entsprechende Verbindungen der Formel

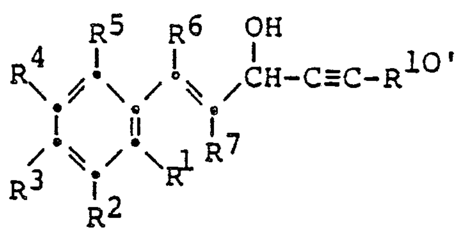

II

worin $R^{10'}$ einen Rest der Formel (a), (b), (d) oder (e) oder einen Rest der Formel

$$-C(R^{18})(R^{19})OR^{20'}$$

(f')

bedeutet;
wobei die übrigen Symbole die in Beschreibung und Ansprüchen definierten Bedeutungen besitzen; haben wertvolle mucosaprotektive und/oder magensäuresekretionshemmende Eigenschaften, so dass sie zur Bekämpfung oder Verhütung von Krankheiten des Magen-Darm-Trakts verwendet werden können, insbesondere gegen Ulcus ventriculi und/oder duodeni.

# Aethinyl-und Aethenyl-Styrylketone

Die vorliegende Erfindung betrifft Styrylketone. Im speziellen betrifft sie Styrylketone der allgemeinen Formel

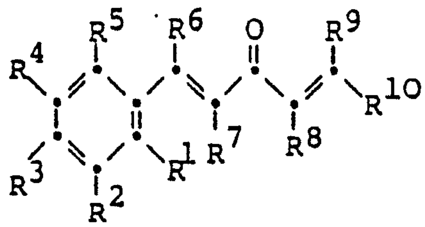

I

worin

$R^1$, $R^2$, $R^3$, $R^4$ und $R^5$ je Wasserstoff, Halogen, niederes Alkyl, niederes Alkoxy-niederes-alkyl, Hydroxy, niederes Alkoxy, niederes Alkenyloxy, niederes Alkinyloxy, niederes Alkoxy-niederes-alkoxy, Acyloxy, Aryl-niederes-alkoxy, niederes Alkylthio, niederes Alkoxy-niederes-alkylthio, niederes Alkenylthio, niederes Alkinylthio, Aryl-niederes-alkylthio, gegebenenfalls substituiertes Amino oder Trifluormethyl oder zwei benachbarte dieser Substituenten gemeinsam und zusammen mit den Kohlenstoffatomen, an welche sie gebunden sind, einen 5-bis 7-gliedrigen Ring bedeuten, wobei von den Substituenten $R^1$ bis $R^5$ mindestens zwei Wasserstoff bedeuten und mindestens einer von Wasserstoff verschieden ist;

$R^6$ und $R^7$ je Wasserstoff oder niederes Alkyl bedeuten;

$R^8$ und $R^9$ je Wasserstoff oder niederes Alkyl oder zusammen eine zusätzliche Kohlenstoff-Kohlenstoff-Bindung bedeuten;

$R^{10}$ einen Rest der Formel

$-COOR^{11}$, (a) $-CONR^{12}R^{13}$, (b) $-C(R^{14})=O$, (c)

$-C(R^{15})(OR^{16})_2$, (d) $-C(OR^{17})_3$ (e) oder

$-C(R^{18})(R^{19})OR^{20}$ (f)

bedeutet;

$R^{11}$ Wasserstoff, niederes Alkyl, niederes Alkenyl, niederes Alkoxy-niederes-alkyl, niederes Alkoxy-niederes-alkoxy-niederes-alkyl, Aryl oder Aryl-niederes-alkyl bedeutet;

$R^{12}$ und $R^{13}$ je Wasserstoff oder niederes Alkyl oder gemeinsam und zusammen mit dem Stickstoffatom einen 5-bis 7-gliedrigen gesättigten heterocyclischen Rest bedeuten;

$R^{14}$ niederes Alkyl, Aryl oder Aryl-niederes-alkyl bedeutet;

$R^{15}$ Wasserstoff, niederes Alkyl, Aryl oder Aryl-niederes-alkyl bedeutet;

$R^{16}$ niederes Alkyl oder niederes Alkoxy-niederes-alkyl bedeutet;

$R^{17}$ niederes Alkyl bedeutet;
$R^{18}$ und $R^{19}$ je Wasserstoff, niederes Alkyl, Aryl oder Aryl-niederes-alkyl bedeuten; und
$R^{20}$ Wasserstoff, niederes Alkyl, niederes Alkoxy-niederes-alkyl, niederes Alkenyl, niederes Alkinyl, Acyl oder Aryl-niederes-alkyl bedeuten;
wobei die im Molekül vorkommende(n) Doppelbindung(en) die E-und/oder Z-Konfiguration aufweist (aufweisen);
sowie pharmazeutisch verwendbare Salze von sauren Verbindungen der Formel I mit Basen und von basischen Verbindungen der Formel I mit Säuren.

Diese Verbindungen sind neu, und es hat sich gezeigt, dass sie wertvolle pharmakodynamische Eigenschaften besitzen, nämlich mucosaprotektive und/oder magensäuresekretionshemmende Eigenschaften, so dass sie zur Bekämpfung oder Verhütung von Krankheiten des Magen-Darm-Trakts verwendet werden können, insbesondere gegen Ulcus ventriculi und/oder duodeni.

Gegenstand der vorliegenden Erfindung sind in erster Linie die eingangs definierten Styrylketone und Salze als solche und als therapeutische Wirkstoffe, die Herstellung dieser Styrylketone und Salze, ferner diese enthaltende Arzneimittel und die Herstellung solcher Arzneimittel, sowie die Verwendung der eingangs definierten Styrylketone und Salze bei der Bekämpfung bzw. Verhütung von Krankheiten, insbesondere bei der Bekämpfung bzw. Verhütung von Ulcus ventriculi und/oder duodeni bzw. die Verwendung der eingangs definierten Styrylketone und Salze sur Herstellung von Arzneimitteln gegen Ulcus ventriculi und/oder duodeni.

Der Ausdruck "nieder" bezeichnet Verbindungen oder Reste mit höchstens 7, vorzugsweise höchstens 4 Kohlenstoffatomen.

Der Ausdruck "Alkyl" bezeichnet geradkettige oder verzweigte gesättigte Kohlenwasserstoffreste, wie Methyl, Aethyl, n-Butyl und dergleichen. Die Ausdrücke "Alkoxy" und "Alkylthio" bezeichnen über ein Sauerstoffatom bzw. ein Schwefelatom gebundene Alkylgruppen im Sinne der vorstehenden Definition, wie Methoxy bzw. Methylthio und dergleichen. Die Ausdrücke "Alkenyl" und "Alkinyl" bezeichnen Kohlenwasserstoffreste, welche eine Kohlenstoff-Kohlenstoff-Doppel-oder -Dreifachbindung enthalten, z.B. also Reste wie Dimethylallyl. Der Ausdruck "Aryl" bezeichnet einen gegebenenfalls substituierten Phenylrest, wie 3,4,5-Trimethoxyphenyl. Der Ausdruck "Acyl" umfasst niedere Alkanoylgruppen, wie Acetyl oder dergleichen, und Aroylgruppen, wie 3,4,5-Trimethoxybenzoyl und dergleichen. Der Ausdruck "Halogen" umfasst die vier Formen Chlor, Fluor, Brom und Jod. Der Ausdruck "gegebenenfalls substituiertes Amino" bezeichnet eine Aminogruppe, die durch niederes Alkyl oder Acyl monosubstituiert oder durch niederes Alkyl und Acyl oder durch zwei niedere Alkylreste disubstituiert sein kann.

Der 5-bis 7-gliedrige Ring, den zwei benachbarte der Substituenten $R^1$, $R^2$, $R^3$, $R^4$, $R^5$ zusammen mit den Kohlenstoffatomen, an welche sie gebunden sind, bilden können, kann heterocyclisch oder carbocyclisch sein, er kann gegebenenfalls eine oder mehrere zusätzliche Doppelbindungen enthalten, wobei er aromatisch oder nicht-aromatisch sein kann, und er kann substituiert oder unsubstituiert sein. Der 5-bis 7-gliedrige gesättigte heterocyclische Rest, den $R^{12}$ und $R^{13}$ zusammen mit dem Stickstoffatom bilden können, kann ein zusätzliches Heteroatom enthalten, und er kann substituiert oder unsubstituiert sein.

In Formel I können beispielsweise $R^1$ und $R^2$ je Wasserstoff und $R^3$, $R^4$ und $R^5$ je niederes Alkoxy oder $R^1$ und $R^5$ je Wasserstoff und $R^2$, $R^3$ und $R^4$ je niederes Alkoxy oder $R^1$, $R^2$ und $R^4$ je Wasserstoff und $R^3$ und $R^5$ je niederes Alkoxy oder $R^1$, $R^3$ und $R^4$ je Wasserstoff und $R^2$ und $R^5$ je niederes Alkoxy oder $R^1$, $R^2$ und $R^5$ je Wasserstoff und $R^3$ Hydroxy und $R^4$ niederes Alkoxy oder $R^3$ und $R^4$ zusammen niederes Alkylendioxy oder $R^1$, $R^2$, $R^4$ und $R^5$ je Wasserstoff und $R^3$ Halogen, niederes Alkoxy, niederes Alkylthio oder Trifluormethyl oder $R^1$, $R^2$, $R^3$ und $R^5$ je Wasserstoff und $R^4$ niederes Alkoxy bedeuten.

Weiterhin können in Formel I $R^{10}$ einen Rest der Formel (a), (d) oder (f), $R^{11}$ Wasserstoff, niederes Alkyl oder niederes Alkoxy-niederes-alkoxy-niederes-alkyl, $R^{15}$ Wasserstoff, $R^{16}$ niederes Alkyl, $R^{18}$ und $R^{19}$ je Wasserstoff und $R^{20}$ Wasserstoff, niederes Alkoxy-niederes-alkyl oder niederes Alkenyl bedeuten.

Vorzugsweise können in Formel I $R^1$, $R^2$, $R^4$ und $R^5$ je Wasserstoff und $R^3$ Fluor oder Methoxy oder $R^1$, $R^2$, $R^3$ und $R^5$ je Wasserstoff und $R^4$ Methoxy bedeuten. $R^6$ und $R^7$ bedeuten vorzugsweise je Wasserstoff; $R^8$ und $R^9$ bedeuten vorzugsweise je Wasserstoff oder zusammen eine zusätzliche Kohlenstoff-Kohlenstoff-Bindung. $R^{10}$ bedeutet vorzugsweise einen Rest der Formel (a) oder (f), wobei vorzugsweise $R^{11}$ Wasserstoff, Methyl oder Methoxyäthoxyäthyl und $R^{20}$ Wasserstoff oder 1-Aethoxyäthyl bedeuten.

Besonders bevorzugte Styrylketone der Formel I sind beispielsweise:
(E,E)-6-(3-Methoxyphenyl)-4-oxo-2,5-hexadiensäure;
(E,E)-6-(4-Methoxyphenyl)-4-oxo-2,5-hexadiensäure;
(2Z,5E)-6-(4-Methoxyphenyl)-4-oxo-2,5-hexadiensäuremethylester und
(E)-6-(4-Methoxyphenyl)-4-oxo-5-hexen-2-insäure.

Weitere bevorzugte Styrylketone der Formel I sind beispielsweise:

(E,E)-6-(4-Fluorphenyl)-4-oxo-2,5-hexadiensäure;
(2Z,5E)-6-(4-Methoxyphenyl)-4-oxo-2,5-hexadiensäure;
(E,E)-6-(3-Methoxyphenyl)-4-oxo-2,5-hexadiensäure-[2-(2-methoxyäthoxy)äthyl]ester; und
(E)-6-(4-Methoxyphenyl)-4-oxo-5-hexen-2-insäure-[2-(2-methoxyäthoxy)äthyl]ester.

Die Styrylketone der eingangs defininerten Formel I und deren Salze können erfindungsgemäss dadurch hergestellt werden, dass man

a) eine Verbindung der allgemeinen Formel

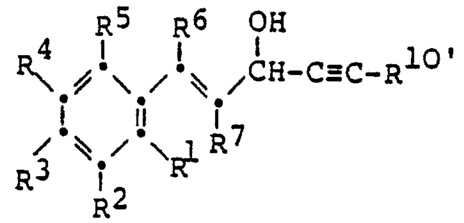

II

worin $R^1$, $R^2$, $R^3$, $R^4$, $R^5$, $R^6$ und $R^7$ obige Bedeutung besitzen und $R^{10'}$ einen Rest der Formel (a), (b), (d) oder (e) oder einen Rest der Formel

$$-C(R^{18})(R^{19})OR^{20'}$$

(f')

bedeutet,

worin $R^{18}$ und $R^{19}$ obige Bedeutung besitzen und $R^{20'}$ die oben für $R^{20}$ definierte Bedeutung besitzt, jedoch nicht Wasserstoff bedeutet, wenn $R^{18}$ und/oder $R^{19}$ Wasserstoff bedeutet,
oxydiert; oder

b) eine Verbindung der obigen Formel II, worin $R^{10'}$ einen Rest der Formel (a) bedeutet, wobei aber $R^{11}$ nicht Wasserstoff bedeutet, mit einer Base behandelt; oder

c) eine Verbindung der allgemeinen Formel

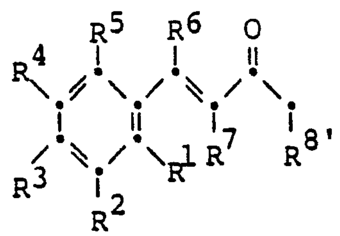

III

worin $R^1$, $R^2$, $R^3$, $R^4$, $R^5$, $R^6$ und $R^7$ obige Bedeutung besitzen und $R^{8'}$ Wasserstoff oder niederes Alkyl bedeutet,
mit einer Verbindung der allgemeinen Formel

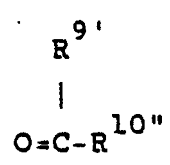

IV

worin $R^{9'}$ Wasserstoff oder niederes Alkyl und $R^{10''}$ einen Rest der Formel (a) bedeuten, wobei $R^{11}$

Wasserstoff bedeutet,
umsetzt; oder

d) eine Verbindung der allgemeinen Formel

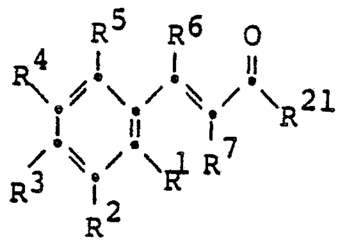

V

worin $R^1$, $R^2$, $R^3$, $R^4$, $R^5$, $R^6$ und $R^7$ obige Bedeutung besitzen und $R^{21}$ eine Abgangsgruppe bedeutet, mit einer Verbindung der allgemeinen Formel

$H{\equiv}C\text{-}R^{10'''}$ VI

worin $R^{10'''}$ einen Rest der Formel (a), (b), (d), (e) oder (f) bedeutet,
umsetzt; oder

e) eine Verbindung der allgemeinen Formel

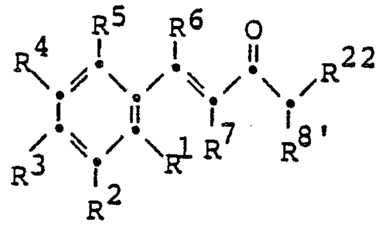

VII

worin $R^1$, $R^2$, $R^3$, $R^4$, $R^5$, $R^6$, $R^7$ und $R^{8'}$ obige Bedeutung besitzen und $R^{22}$ Halogen bedeutet, mit einer Verbindung der allgemeinen Formel

$(R^{23})_3P=CH\text{-}R^{10iv}$ VIII

worin $R^{23}$ einen Arylrest und $R^{10iv}$ einen Rest der Formel (a), (b), (d), (e) oder (f) bedeutet, wobei aber $R^{11}$ und $R^{20}$ nicht Wasserstoff bedeuten,
umsetzt; oder

f) von einer Verbindung der allgemeinen Formel

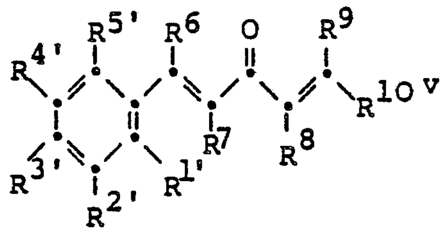

IX

worin $R^{1'}$, $R^{2'}$, $R^{3'}$, $R^{4'}$ und $R^{5'}$ die oben für $R^1$, $R^2$, $R^3$, $R^4$ und $R^5$ angegebene Bedeutung besitzen und maximal drei davon zusätzlich geschütztes Hydroxy, geschütztes Amino oder geschütztes niederes Alkylamino bedeuten können, $R^6$, $R^7$, $R^8$ und $R^9$ obige Bedeutung besitzen, $R^{10v}$ einen Rest der Formel (a), (b), (c), (d), (e) oder

$$-C(R^{18})(R^{19})OR^{20''}$$

(f")

worin $R^{18}$ und $R^{19}$ obige Bedeutung besitzen und $R^{20''}$ die oben für $R^{20}$ angegebene Bedeutung besitzt und zusätzlich eine Schutzgruppe bedeuten kann, wobei das Molekül mindestens eine Schutzgruppe enthält,
die Schutzgruppe(n) abspaltet; oder

g) eine Verbindung der Formel I, worin $R^{10}$ einen Rest der Formel (a) bedeutet, wobei $R^{11}$ von Wasserstoff verschieden ist, zur entsprechenden Carbonsäure hydrolysiert; oder

h) eine Verbindung der Formel I, worin $R^{10}$ einen Rest der Formel (f) bedeutet, wobei $R^{20}$ Wasserstoff bedeutet, acyliert; oder

i) eine Verbindung der Formel I, worin $R^8$ und $R^9$ zusammen eine zusätzliche Kohlenstoff-Kohlenstoff-Bindung und $R^{10}$ einen Rest der Formel (d) bedeutet, wobei $R^{15}$ Wasserstoff bedeutet, in die entsprechende Verbindung der Formel I, worin $R^8$ und $R^9$ je Wasserstoff, $R^{10}$ einen Rest der Formel (a) und $R^{11}$ Wasserstoff bedeuten, überführt; oder

j) eine Verbindung der Formel I, worin $R^{10}$ einen Rest der Formel (d) bedeutet, in die entsprechende Verbindung der Formel I, worin $R^{10}$ einen Rest der Formel (c) bedeutet, überführt; oder

k) eine saure Verbindung der Formel I mit einer Base bzw. eine basische Verbindung der Formel I mit einer Säure in ein pharmazeutisch verwendbares Salz überführt.

Die Verbindungen der obigen Formel II sind neu und ebenfalls Gegenstand der vorliegenden Erfindung, ebenso auch ein Verfahren zu ihrer Herstellung, welches dadurch gekennzeichnet ist, dass man eine Verbindung der allgemeinen Formel

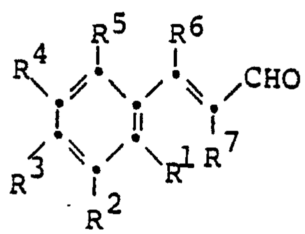

X

worin $R^1$, $R^2$, $R^3$, $R^4$, $R^5$, $R^6$ und $R^7$ obige Bedeutung besitzen,
mit einer Verbindung der allgemeinen Formel

$HC{\equiv}C{-}R^{10vi}$ XI

worin $R^{10vi}$ einen Rest der Formel (a), (b), (d), (e) oder (f') bedeutet,
umsetzt.

Die Verbindungen der allgemeinen Formel II haben ähnliche pharmakodynamische Eigenschaften wie die Styrylketone der Formel I, und zwar vor allem diejenigen, worin $R^{10'}$ einen Rest der Formel (a) bedeutet, insbesondere diejenigen, worin $R^{11}$ niederes Alkyl oder niederes Alkoxy-niederes-alkoxy-niederes-alkyl bedeutet; repräsentative Beispiele derartiger Verbindungen der Formel II sind der (E)-4-Hydroxy-6-(4-methoxyphenyl)-5-hexen -2-insäuremethylester und der (E)-4-Hydroxy-6-(4-methoxyphenyl)-5-hexen-2-insäure-[2-(2-methoxyäthoxy)äthyl]ester.

Gegenstand der vorliegenden Erfindung sind demnach auch Verbindungen der allgemeinen Formel II als therapeutische Wirkstoffe, diese enthaltende Arzneimittel und die Herstellung solcher Arnzneimittel, sowie die Verwendung der Verbindungen der Formel II bei der Bekämpfung bzw. Verhütung von Krankheiten, insbesondere bei der Bekämpfung bzw. Verhütung von Ulcus ventriculi und/oder duodeni bzw. zur Herstellung von Arzneimitteln gegen Ulcus ventriculi und/oder duodeni.

Verfahrensvariante a) gemäss vorliegender Erfindung liefert Styrylketone der Formel I, worin $R^8$ und $R^9$ zusammen eine zusätzliche Kohlenstoff-Kohlenstoff-Bindung und $R^{10}$ einen Rest der Formel (a), (b), (d), (e) oder (f') bedeuten.

Die Oxidation gemäss Verfahrensvariante a) erfolgt nach an sich bekannten Methoden, welche jedem Fachmann, der sich die Aufgabe stellt, eine Hydroxygruppe in eine Oxogruppe überzuführen, geläufig sind. Als Oxidationsmittel verwendet man zweckmässigerweise Mangandioxid (Braunstein) in einem hierfür geeigneten, unter den Reaktionsbedingungen inerten Lösungsmittel, beispielsweise in einem halogenierten Kohlenwasserstoff, wie Methylenchlorid oder dergleichen. Die Oxidation mittels Mangandioxid erfolgt zweckmässigerweise in einem Temperaturbereich von etwa 0° bis etwa Raumtemperatur und dauert, je nach den übrigen Bedingungen, ca. 10 Minuten bis ca. 20 Stunden.

Verfahrensvariante b) gemäss vorliegender Erfindung liefert Styrylketone der Formel I, worin $R^8$ und $R^9$ je Wasserstoff und $R^{10}$ einen Rest der Formel (a) bedeuten, wobei aber $R^{11}$ von Wasserstoff verschieden ist. Als Base verwendet man zweckmässigerweise eine bicyclische Stickstoffverbindung, wie 1,8-Diazabicyclo-

[5.4.0]undec-7-en, 1,9-Diazabicyclo[4.3.0]non-5-en oder dergleichen. Zweckmässigerweise erfolgt die Reaktion in einem unter den Reaktionsbedingungen inerten organischen Lösungsmittel, beispielsweise in einem halogenierten Kohlenwasserstoff, wie Methylenchlorid, oder dergleichen. Die Reaktionstemperatur liegt zweckmässigerweise in einem Bereich um 0°C, und die Reaktionsdauer beträgt etwa 20 bis etwa 50 (beispielsweise etwa 35) Stunden.

Der erfindungsgemässe Verfahrensaspekt (c) liefert Styrylketone der Formel I, worin $R^8$ und $R^9$ je Wasserstoff oder niederes Alkyl und $R^{10}$ einen Rest der Formel (a) bedeuten, wobei $R^{11}$ Wasserstoff bedeutet, d.h. also Carbonsäuren. Als Verbindung der Formel IV verwendet man beispielsweise Glyoxylsäure, welche zweckmässigerweise in Form des Monohydrats eingesetzt wird. Die Umsetzung der Verbindungen der Formeln III und IV erfolgt zweckmässigerweise unter sauren Bedingungen, beispielsweise in Essigsäure oder dergleichen. Die Reaktion wird zweckmässigerweise bei erhöhter Temperatur durchgeführt, beispielsweise bei Rückflusstemperatur des verwendeten Reaktionssystems, und sie dauert ca. 10 bis ca. 30 (beispielsweise ca. 20) Stunden.

Der erfindungsgemässe Verfahrensaspekt (d) liefert Styrylketone der Formel I, worin $R^8$ und $R^9$ zusammen eine zusätzliche Kohlenstoff-Kohlenstoff-Bindung und $R^{10}$ einen Rest der Formel (a), (b), (d), (e) oder (f) bedeutet. Als Abgangsgruppen ($R^{21}$) in den als Ausgangsprodukte verwendeten Verbindungen der Formel V eignen sich Reste, wie N-Methoxy-N-methylamino oder dergleichen. Die Umsetzung der Verbindungen der Formeln V und VI erfolgt in Gegenwart einer starken Base, wie n-Butyllithium, Alkylmagnesiumhalogenide (z.B. Aethylmagnesiumbromid) oder dergleichen, in einem unter den Reaktionsbedingungen inerten organischen Lösungsmittel oder Lösungsmittelgemisches, wie Tetrahydrofuran/n-Hexan oder dergleichen. Die Reaktionstemperatur richtet sich natürlich unter anderem nach der verwendeten Base und nach dem verwendeten Lösungsmittelsystem, und sie liegt zweckmässigerweise in einem Bereich von etwa -100°C bis etwa 0°C. Die Reaktionsdauer beträgt etwa 5 bis etwa 60 (beispielsweise etwa 10 bis etwa 30) Minuten.

Verfahrensaspekt e) gemäss vorliegender Erfindung liefert Styrylketone der Formel I, worin $R^8$ und $R^9$ je Wasserstoff und $R^{10}$ einen Rest der Formel (a), (b), (d), (e) oder (f) bedeuten, wobei aber $R^{11}$ bzw. $R^{20}$ von Wasserstoff verschieden sind. Die Umsetzung der Verbindungen der Formeln VII und VIII erfolgt zweckmässigerweise in einem unter den Reaktionsbedingungen inerten organischen Lösungsmittel, beispielsweise in einem aromatischen Kohlenwasserstoff, wie Toluol oder dergleichen, usw. Die Reaktionstemperatur hängt unter anderem von der Natur der als Ausgangsprodukte eingesetzten Verbindungen der Formeln VII und VIII sowie des verwendeten Lösungsmittels ab; zweckmässigerweise arbeitet man bei erhöhter Temperatur, beispielsweise bei Rückflusstemperatur des Reaktionssystems. Die Reaktionsdauer beträgt ca. 1 bis ca. 5 (beispielsweise 2) Stunden.

Als Schutzgruppen in den Verbindungen der allgemeinen Formel IX, welche als Ausgangsprodukte in Verfahrensvariante f) verwendet werden, eignen sich selbstverständlich nur solche, welche durch Methoden abgespalten werden können, bei welchen diese Schutzgruppen selektiv entfernt werden, ohne dass andere im Molekül vorhandene Strukturelemente in Mitleidenschaft gezogen werden. Die Entfernung der Schutzgruppe bzw. der Schutzgruppen aus den Verbindungen der allgemeinen Formel IX erfolgt nach an sich bekannten Methoden, wobei natürlich für die Wahl der zur Anwendung gelangenden Methode die Natur der zu entfernenden Schutzgruppe bzw. Schutzgruppen in Betracht gezogen werden muss und zu beachten ist, dass nur die Schutzgruppe bzw. Schutzruppen selektiv entfernt, andere im Molekül vorhandene Strukturelemente jedoch nicht in Mitleidenschaft gezogen werden sollen. Als O-Schutzgruppen eignen sich beispielsweise leicht abspaltbare Acetal-und Ketalschutzgruppen, wie Methoxymethyl, Methoxyäthoxymethyl, 1-Aethoxyäthyl, 2-(Trimethylsilyl)äthoxymethyl, Tetrahydro-2H-pyran-2-yl und dergleichen; leicht abspaltbare metallorganische Gruppen, insbesondere Trialkylsilylgruppen, wie Trimethylsilyl, t-Butyldimethylsilyl und dergleichen; leicht abspaltbare Aralkylgruppen, wie Triphenylmethyl und dergleichen; leicht abspaltbare Acylgruppen, wie Acetyl und dergleichen; usw. Als N-Schutzgruppen eignen sich in erster Linie leicht abspaltbare Acylgruppen, wie t-Butyloxycarbonyl und dergleichen.

Methoden für die Entfernung der Reste, welche vorstehend als Beispiele für Schutzgruppen erwähnt wurden, sind in der Literatur beschrieben und demnach jedem Fachmann geläufig. So kann man beispielsweise die Methoxymethylgruppe, die Methoxyäthoxymethylgruppe, die 1-Aethoxyäthylgruppe, die 2-(Trimethylsilyl)äthoxymethylgruppe, die Tetrahydro-2H-pyran-2-ylgruppe, die Trimethylsilylgruppe, die t-Butyldimethylsilylgruppe und die Triphenylmethylgruppe unter sauren Bedingungen abspalten, beispielsweise mittels wässriger Salzsäure in einem unter den Reaktionsbedingungen inerten organischen Lösungsmittel, wie Tetrahydrofuran, die Tetrahydro-2H-pyran-2-ylgruppe, die Trimethylsilylgruppe und die t-Butyldimethylsilylgruppe aber zweckmässigerweise auch mittels Pyridinium-p-toluolsulfonat in einem unter den Reaktionsbedingungen inerten organischen Lösungsmittel oder Lösungsmittelgemisch, wie Tetrahydrofuran/Aethanol, und die Trimethylsilylgruppe und die t-Butyldimethylsilylgruppe auch mittels

eines quartären Ammoniumfluorids, wie Tetrabutylammoniumfluorid, in einem unter den Reaktionsbedingungen inerten organischen Lösungsmittel, wie Tetrahydrofuran. Die Acetylgruppe kann unter milden alkalischen Bedingungen abgespalten werden, beispielsweise mittels verdünnter (ca. 2-5%iger) Kalilauge in einem unter den Reaktionsbedingungen inerten organischen Lösungsmittel, wie Tetrahydrofuran. Die Abspaltung einer t-Butyloxycarbonylgruppe kann unter sauren Bedingungen erfolgen, z.B. mittels einer wässrigen Säure oder mittels wasserfreier Trifluoressigsäure.

Die Hydrolyse gemäss Verfahrensvariante (g) erfolgt nach an allgemein bekannten und jedem Fachmann geläufigen Methoden, zweckmässigerweise mittels einer starken anorganischen Base, beispielsweise eines Alkalimetallhydroxids, wie Kaliumhydroxid oder dergleichen, in einem geeigneten Lösungsmittelsystem, beispielsweise in Wasser oder wässrigem Tetrahydrofuran und dergleichen.

Auch die Acylierung gemäss Verfahrensvariante (h) erfolgt nach allgemein üblichen Methoden. Als Acylierungsmittel verwendet man beispielsweise ein dem einzuführenden Acylrest entsprechendes Säurehalogenid, wie Acetylchlorid, 3,4,5-Trimethoxybenzoylchlorid und dergleichen. Die Acylierung mittels eines derartigen Säurehalogenids erfolgt zweckmässigerweise in Gegenwart einer Base, insbesondere einer tertiären organischen Base, wie Pyridin, Triäthylamin, N-Methylpiperidin, 4-Dimethylaminopyridin oder dergleichen. Als Lösungsmittel eignen sich in erster Linie Halogenierte Kohlenwasserstoffe, wie Methylenchlorid oder dergleichen; falls als Base Pyridin verwendet wird, so kann dieses gleichzeitig auch als Lösungsmittels dienen. Bedeuten eines oder mehrere von $R^1$-$R^5$ eine Hydroxygruppe und/oder eine Aminogruppe und/oder eine niedere Alkylaminogruppe, so wird bzw. werden diese ebenfalls acyliert.

Verfahrensvariante i) gemäss vorliegender Erfindung erfolgt unter sauren Bedingungen, zweckmässigerweise in Gegenwart von wässriger Säure, beispielsweise wässriger Bromwasserstoffsäure oder dergleichen, in einem unter den Reaktionsbedingungen inerten, mit Wasser mischbaren organischen Lösungsmittel, wie Dioxan oder dergleichen. Die Reaktionstemperatur beträgt zweckmässigerweise etwa 10 bis etwa 50°C (beispielsweise etwa 30°C), und die Reaktionszeit beträgt in der Regel ca. 10 bis ca. 30 (beispielsweise ca. 18) Stunden.

Gemäss Verfahrensvariante (j) wird eine Acetal-oder Ketalgruppe in eine Carbonylgruppe übergeführt. Dies erfolgt nach allgemein üblichen und jedem Fachmann geläufigen Methoden, zweckmässigerweise mittels wässriger Perchlorsäure oder dergleichen, in einem unter den Reaktionsbedingungen inerten organischen Lösungsmittel, wie Dioxan oder dergleichen, bei etwa Raumtemperatur und dauert einige (bei spielsweise 2) Stunden.

Die Ueberführung einer sauren Verbindung der Formel I in ein pharmazeutisch verwendbares Salz kann durch Behandeln mit einer geeigneten Base in an sich bekannter Weise bewerkstelligt werden. Als derartige Salze eignen sich sowohl solche mit von einer anorganischen Base abgeleiteten Kationen, z.B. also Kaliumsalze, Natriumsalze, Calciumsalze und dergleichen, als auch Salze mit organischen Basen, wie Aethylendiamin, Monoäthanolamin, Diäthanolamin und dergleichen.

Die Ueberführung einer basischen Verbindung der Formel I in ein pharmazeutisch verwendbares Salz kann durch Behandeln mit einer geeigneten Säure bewerkstelligt werden. Als derartige Salze eignen sich sowohl solche mit anorganischen Säuren, wie Chlorwasserstoff, Bromwasserstoff, Phosphorsäure, Schwefelsäure und dergleichen, als auch Salze mit organischen Säuren, wie Zitronensäure, Aepfelsäure, Methansulfonsäure, p-Toluolsulfonsäure und dergleichen.

Die erfindungsgemässe Herstellung von Verbindungen der Formel II aus Verbindungen der Formel X und XI erfolgt in Gegenwart einer starken Base, wie n-Butyllithium, Alkylmagnesiumhalogenide (z.B. Aethylmagnesiumbromid) oder dergleichen, in einem unter den Reaktionsbedingungen inerten organischen Lösungsmittel oder Lösungsmittelgemisch, wie Tetrahydrofuran/n-Hexan und dergleichen. Die Reaktionstemperatur richtet sich unter anderem nach der Natur der eingesetzten Ausgangsprodukte der Formeln X und XI, der verwendeten Base und des verwendeten Lösungsmittels bzw. Lösungsmittelgemisches; sie liegt im allgemeinen in einem Bereich von etwa -110 bis etwa 0°C, insbesondere von etwa -80 bis -70 (beispielsweise bei etwa 78) °C. Die Reaktionsdauer kann in Abhängigkeit von den übrigen Reaktionsparametern einige Minuten bis einige Stunden betragen, bei spielsweise ca. 5 Minuten bis ca. 2 Stunden.

Die Ausgangsprodukte der Formel III können beispielsweise durch Umsetzung von Verbindungen der Formel

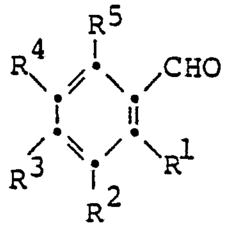

XII

worin $R^1$, $R^2$, $R^3$, $R^4$ und $R^5$ obige Bedeutung besitzen,
mit Verbindung der allgemeinen Formel

$R^7$-$CH_2$-CO-$CH_2$-$R^{8'}$ XIII

worin $R^7$ und $R^{8'}$ obige Bedeutung besitzen,
nach an sich bekannten Methoden hergestellt werden. Verschiedene der nachfolgenden Beispiele enthalten überdies detaillierte Angaben betreffend die Herstellung bestimmter Verbindungen der Formel III.

Die Ausgangsprodukte der Formel IV sind bekannt oder nach an sich bekannten und jedem Fachmann geläufigen Methoden leicht herstellbar.

Ausgangsprodukte der Formel V können nach an sich bekannten und jedem Fachmann geläufigen Methoden aus entsprechenden Verbindungen der Formel

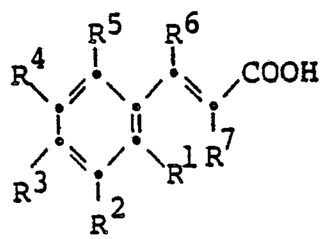

XIV

worin $R^1$, $R^2$, $R^3$, $R^4$, $R^5$, $R^6$ und $R^7$ obige Bedeutung besitzen,
hergestellt werden. Verbindungen der Formel V, worin $R^{21}$ N-Methoxy-N-methylamino bedeutet, erhält man beispielsweise durch Umsetzung von Verbindungen der Formel XIV mit N,O-Dimethylhydroxylamin in Gegenwart von Dicyclohexylcarbodiimid oder dergleichen in einem unter den Reaktionsbedingungen inerten organischen Lösungsmittel, beispielsweise einem halogenierten Kohlenwasserstoff, wie Methylenchlorid oder dergleichen, usw. Zudem enthalten verschiedene der nachfolgenden Beispiele detaillierte Angaben betreffend die Herstellung bestimmter Verbindungen der Formel V.

Die Ausgangsprodukte der Formel VI sind bekannt oder nach an sich bekannten und jedem Fachmann geläufigen Methoden leicht zugänglich; zudem enthalten verschiedene der nachfolgenden Beispiele detaillierte Angaben betreffend die Herstellung bestimmter Verbindungen der Formel VI.

Verbindungen der Formel VII können beispielsweise ausgehend von entsprechenden Verbindungen der Formel III durch Umsetzung mit einem geeigneten Halogenierungsmittel, wie 5,5-Dibrombarbitursäure oder dergleichen, hergestellt werden.

Verbindungen der Formel VIII können beispielsweise dadurch hergestellt werden, dass man eine Verbindung der Formel

$R^{22}$-$CH_2$-$R^{10iv}$ XV

worin $R^{22}$ und $R^{10iv}$ obige Bedeutung besitzen, mit einem Triarylphosphin, wie Triphenylphosphin oder dergleichen, umsetzt (beispielsweise in einem aromatischen Kohlenwasserstoff, wie Toluol oder dergleichen, bei etwa Raumtemperatur, was ca. 4 Stunden dauert), worauf man durch Behandlung des erhaltenen Phosphoniumhalogenids mit einer Base (z.B. mit wässriger Natriumhydroxidlösung oder dergleichen) das gewünschte entsprechende Phosphoran der Formel VIII erhält.

Die Herstellung von Verbindungen der Formel IX kann in Analogie zur Herstellung entsprechender Styrylketone der Formel I erfolgen.

Die Verbindungen der Formeln X, XI, XII, XIII, XIV und XV sind bekannt oder nach an sich bekannten und jedem Fachmann geläufigen Methoden leicht herstellbar.

Wie oben erwähnt, besitzen die Verbindungen der allgemeinen Formeln I und II sowie pharmazeutisch verwendbare Salze von Verbindungen der Formel I wertvolle pharmakodynamische Eigenschaften.

Repräsentative Verbindungen der Formeln I und II wurden auf ihre mucosaprotektiven und magnesäuresekretionshemmenden Eigenschaften sowie auf ihre Toxizität untersucht.

Zur Bestimmung der muscosa-protektiven Eigenschaft wurde die nachstehend beschriebene Versuchsanordnung verwendet:

Orale Verabreichung von absolutem Aethanol an männliche Ratten in einer Dosis von 1 ml pro Ratte führt innerhalb 1 Stunde zu blutigen Läsionen der Magenschleimhaut. Verschiedene Dosen der zu prüfenden Substanzen (suspendiert in 0,125% Carboxymethylcellulose) oder das Vehikel allein (Kontrolle) werden den Ratten oral (1 ml pro Ratte) 20 Minuten vor der Behandlung mit Aethanol verabreicht. Eine Stunde nach der Verabreichung des Aethanols tötet man die Tiere, untersucht deren Mägen auf das Vorhandensein von Läsionen und ermittelt die Anzahl und die gesamte Ausdehnung solcher Läsionen. Als $ID_{50}$ bezeichnet man diejenige Dosis einer Testsubstanz, welche die Anzahl der Läsionen im Vergleich zur Kontrollgruppe um 50% reduziert.

Zur Bestimmung der magensäuresekretionshemmenden Wirkung wurde die nachstehend beschriebene Versuchsanordnung verwendet:

Männlichen Ratten wird unter leichter Aethernarkose gemäss Shay et al. [Gastroenterology 5, 43 (1945)] der Pylorus ligiert. Die zu prüfenden Substanzen, suspendiert in 0,5% Carboxymethylcellulose, werden intraduodenal verabreicht; Kontrolltiere werden lediglich mit dem Vehikel behandelt. Fünf Stunden nach der Ligation tötet man die Tiere, bestimmt das Volumen und die Azidität ihres Magensaftes und vergleicht die erhaltenen Werte mit denjenigen von Kontrolltieren. Als $ID_{50}$ bezeichnet man diejenige Dosis einer Testsubstanz, welche bei den behandelten Tieren im Vergleich zu den Kontrolltieren eine 50%ige Verminderung der Sekretion bewirkt.

In der nachfolgenden Tabelle werden für eine Reihe repräsentativer Verbindungen der Formel I und für zwei Verbindungen der Formel II die Resultate der Prüfung auf ihre mucosa-protektive Wirkung ("Aethanol-Test") und auf ihre magensäuresekretionshemmende Wirkung wiedergegeben. Ausserdem enthält diese Tabelle Angaben über die akute Toxizität ($DL_{50}$ bei einmaliger oraler Verabreichung an Mäuse).

| Verbindung | Aethanol-Test, ID 50 mg/kg p.o. | Magensäuresekretionshemmung, ID 50 mg/kg i.d. | Toxizität, DL 50 mg/kg p.o. |
|---|---|---|---|
| A | 0,8 | 9 | 312- 625 |
| B | 0,7 | - | 312- 625 |
| C | 1,0 | - | 625-1250 |
| D | 0,5 | - | 500-1000 |
| E | 1,9 | - | 312- 625 |

| | | | |
|---|---|---|---|
| F | 0,7 | - | 156- 312 |
| G | 2,7 | - | 500-1000 |
| H | 2,7 | - | 2500-5000 |
| I | 2,1 | - | 1000-2000 |
| J | 3,4 | - | >4000 |

A = (E,E)-6-(3-Methoxyphenyl)-4-oxo-2,5-hexadiensäure
B = (E,E)-6-(4-Methoxyphenyl)-4-oxo-2,5-hexadiensäure
C = (2Z,5E)-6-(4-Methoxyphenyl)-4-oxo-2,5-hexadiensäure-methylester
D = (E)-6-(4-Methoxyphenyl)-4-oxo-5-hexen-2-insäure
E = (E,E)-6-(4-Fluorphenyl)-4-oxo-2,5-hexadiensäure
F = (2Z,5E)-6-(4-Methoxyphenyl)-4-oxo-2,5-hexadiensäure
G = (E,E)-6-(3-Methoxyphenyl)-4-oxo-2,5-hexadiensäure-[2-(2-methoxyäthoxy)äthyl]ester
H = (E)-6-(4-Methoxyphenyl)-4-oxo-5-hexen-2-insäure-[2-(2-methoxyäthoxy)äthyl]ester
I = (E)-4-Hydroxy-6-(4-methoxyphenyl)-5-hexen-2-insäure-methylester
J = (E)-4-Hydroxy-6-(4-methoxyphenyl)-5-hexen-2-insäure-[2-(2-methoxyäthoxy)äthyl]ester

Die Verbindungen der Formeln I und II und die pharmazeutisch verwendbaren Salze von Verbindungen der Formel I können als Heilmittel, z.B. in Form pharmazeutischer Präparate Verwendung finden. In erster Linie kommt orale Verabreichung in Form von festen pharmazeutischen Präparaten, wie Tabletten, Lacktabletten, Dragées, Hartgelatinekapseln und Weichgelatinekapseln in Frage. Orale Verabreichung in Form flüssiger pharmazeutischer Präparate, wie Lösungen, Emulsionen und Suspensionen, rektale Verabreichung, z.B. in Form von Suppositorien, oder parenterale Verabreichung, z.B. in Form von Injektionslösungen, sind ebenfalls in Betracht zu ziehen.

Arzneimittel, enthaltend eine Verbindung der Formel I oder II oder ein pharmazeutisch verwendbares Salz einer Verbindung der Formel I, sind ebenfalls Gegenstand der vorliegenden Erfindung. Die Herstellung derartiger Arzneimittel kann dadurch erfolgen, dass man eine oder mehrere der Verbindungen der Formeln I oder II oder der pharmazeutisch verwendbaren Salze der Verbindungen der Formel I und erwünschtenfalls einen oder mehrere andere therapeutische Wirkstoffe zusammen mit einem oder mehreren therapeutisch inerten Excipientien in eine galenische Darreichungsform bringt.

Zur Herstellung von Tabletten, Lacktabletten, Dragées und Hartgelatinekapseln können die Verbindungen der Formeln I und II und die pharmazeutisch verwendbaren Salze von Verbindungen der Formel I mit pharmazeutisch inerten, anorganischen oder organischen Excipientien verarbeitet werden. Als solche Excipientien kann man z.B. für Tabletten, Dragées und Hartgelatinekapseln Lactose, Maisstärke oder Derivate davon, Talk, Stearinsäure oder deren Salze etc. verwenden. Zur Herstellung von magensaftresistenten pharmazeutischen Präparaten ist noch ein magensaftresistenter Lack aufzubringen, welcher z.B. aus Hydroxypropylmethylcellulosephthalat bestehen kann.

Für Weichgelatinekapseln eignen sich als Excipientien z.B. vegetabile Oele, Wachse, Fette, halbfeste und flüssige Polyole etc.

Zur Herstellung von Lösungen und Sirupen eignen sich als Excipientien z.B. Wasser, Polyole, Saccharose, Invertzucker, Glucose und dgl.

Für Suppositorien eignen sich als Excipientien z.B. natürliche oder gehärtete Oele, Wachse, Fette, halbflüssige oder flüssige Polyole und dgl.

Für Injektionslösungen eignen sich als Excipientien z.B. Wasser, Alkohole, Polyole, Glycerin, vegetabile Oele etc.

Die pharmazeutischen Präparate können daneben noch Konservierungsmittel, Lösevermittler, Stabilisierungsmittel, Netzmittel, Emulgiermittel, Süssmittel, Färbemittel, Aromatisierungsmittel, Salze zur Veränderung des osmotischen Druckes, Puffer, Ueberzugsmittel oder Antioxidantien enthalten. Sie können auch noch andere therapeutisch wertvolle Stoffe enthalten.

Erfindungsgemäss kann man die Verbindungen der Formeln I und II und die Pharmazeutisch verwendbaren Salze von Verbindungen der Formel I bei der Bekämpfung bzw. Verhütung von Krankheiten verwenden, beispielsweise bei der Bekämpfung bzw. Verhütung von Ulcus ventriculi und/oder duodeni. Die Dosierung kann innerhalb weiter Grenzen variieren und ist natürlich in jedem einzelnen Fall den individuellen Gegebenheiten anzupassen. Im allgemeinen dürfte bei oraler Verabreichung eine Tagesdosis von etwa 30-400 mg und bei intravenöser Verabreichung eine Tagesdosis von etwa 1-50 mg angemessen sein.

Gegenstand der Erfindung ist auch die Verwendung der Verbindungen der Formeln I und II und der pharmazeutisch verwendbaren Salze von Verbindungen der Formel I zur Herstellung von Arzneimitteln gegen Ulcus ventriculi und/oder duodeni.

In den nachfolgenden Beispielen, welche die vorliegende Erfindung illustrieren, ihren Umfang aber in keiner Weise beschränken sollen, sind sämtliche Temperaturen in Celsiusgraden angegeben.

## Beispiel 1

a) Eine Lösung von 19,6 g (0,1 Mol) 2,3,4-Trimethoxybenzaldehyd in 21 ml (0,28 Mol) Aceton und 10,5 ml Wasser wird mit 2,5 ml 3N Natronlauge versetzt, wobei die Temperatur des Reaktionsgemisches 30° nicht übersteigen soll. Man rührt das Gemisch 20 Stunden bei Raumtemperatur, verdünnt mit Wasser und setzt bis zur stark sauren Reaktion 3N Salzsäure zu. Die wässrige Phase wird zweimal mit Methylenchlorid extrahiert; die vereinigten organischen Phasen werden mit Wasser gewaschen, über Magnesiumsulfat getrocknet und eingeengt. Der Rückstand wird durch Flash-Chromatographie an 1000 g Silicagel (Elutionsmittel Aether/Hexan 10:1) gereinigt. Man erhält (E)-4-(2,3,4-Trimethoxyphenyl)but-3-en-2-on als gelbes Oel.

MS: 236 ($M^+$), 205 (base peak) m/e.

b) Eine Lösung von 18 g (76 mMol) (E)-4-(2,3,4-Trimethoxyphenyl)but-3-en-2-on und 7 g (76 mMol) Glyoxylsäure-monohydrat in 19 ml Essigsäure wird während 20 Stunden am Rückfluss gekocht. Das Reaktionsgemisch wird mit Wasser verdünnt und mit Essigester extrahiert. Die organische Phase wird zweimal mit 3N Natronlauge extrahiert; die vereinigten wässrigen Phasen werden bis zur stark sauren Reaktion mit 3N Salzsäure versetzt und zweimal mit Essigester extrahiert. Die vereinigten organischen Phasen werden über Magnesiumsulfat getrocknet und eingeengt. Kristallisation des Rückstandes aus Aether/Methylenchlorid liefert (E,E)-4-Oxo-6-(2,3,4-trimethoxyphenyl)-2,5-hexadiensäure vom Schmelzpunkt 125-127°.

## Beispiel 2

Eine Lösung von 21,5 g (131 mMol) (E)-4-(4-Fluorphenyl) but-3-en-2-on und 12 g (131 mMol) Glyoxylsäure-monohydrat in 32 ml Essigsäure wird während 20 Stunden am Rückfluss gekocht. Das Reaktionsgemisch wird mit Wasser verdünnt und mit essigester extrahiert. Die organische Phase wird zweimal mit 3N Natronlauge extrahiert; die vereinigten wässrigen Phasen werden bis zur stark sauren Reaktion mit 3N Salzsäure versetzt und zweimal mit Essigester extrahiert. Die vereinigten organischen Phasen werden über Magnesiumsulfat getrocknet und eingeengt. Kristallisation des Rückstandes aus Essigester/Aether liefert (E,E)-6-(4-Fluorphenyl)-4-oxo-2,5-hexadiensäure vom Schmelzpunkt 160-162°.

Beispiel 3

Eine Lösung von 10,5 g (54,6 mMol) (E)-4-[4-(Methylthio)phenyl]but-3-en-2-on und 5,02 g (54,6 mMol) Glyoxylsäure-monohydrat in 15 ml Essigsäure wird während 20 Stunden am Rückfluss gekocht. Das Reaktionsgemisch wird mit Wasser verdünnt und mit Methylenchlorid extrahiert. Die organische Phase wird zweimal mit 3N Natronlauge extrahiert; die vereinigten wässrigen Phasen werden bis zur stark sauren Reaktion mit 3N Salzsäure versetzt und zweimal mit Methylenchlorid extrahiert. Die vereinigten organischen Phasen werden über Magnesiumsulfat getrocknet und eingeengt. Kristallisation des Rückstandes aus Methylenchlorid/Aether liefert (E,E)-6-[4-(Methythio)phenyl]-4-oxo-2,5-hexadiensäure vom Schmelzpunkt 160-163°.

Beispiel 4

Eine Lösung von 19 g (92 mMol) (E)-4-(2,4-Dimethoxyphenyl)but-3-en-2-on und 8,4 g (92 mMol) Glyoxylsäure-monohydrat in 24 ml Essigsäure wird während 20 Stunden am Rückfluss gekocht. Das Reaktionsgemisch wird mit Wasser verdünnt und mit Essigester extrahiert. Die organische Phase wird zweimal mit 3N Natronlauge extrahiert; die vereinigten wässrigen Phasen werden bis zur stark sauren Reaktion mit 3N Salzsäure versetzt und zweimal mit Essigester extrahiert. Die vereinigten organischen Phasen werden über Magnesiumsulfat getrocknet und eingeengt. Kristallisation des Rückstandes aus Methylenchlorid/Aether/Hexan liefer (E,E)-6-(2,4-Dimethoxyphenyl)-4-oxo-2,5-hexadiensäure vom Schmelzpunkt 195-200°.

Beispiel 5

Eine Lösung von 17 g (96 mMol) (E)-4-(4-Methoxyphenyl)but-3-en-2-on und 8,9 g (96 mMol) Glyoxylsäure-monohydrat in 25 ml Essigsäure wird während 20 Stunden am Rückfluss gekocht. Das Reaktionsgemisch wird mit Wasser verdünnt und mit Essigester extrahiert. Die organische Phase wird zweimal mit 3N Natronlauge extrahiert; die vereinigten wässrigen Phasen werden bis zur stark sauren Reaktion mit 3N Salzsäure versetzt und zweimal mit Essigester extrahiert. Die vereinigten organischen Phasen werden über Magnesiumsulfat getrocknet und eingeengt. Kristallisation des Rückstandes aus Methylenchlorid/Aether/Hexan liefert (E,E)-6-(4-Methoxyphenyl)-4-oxo-2,5-hexadiensäure vom Schmelzpunkt 138-139°.

Beispiel 6

Eine Lösung von 14,7 g (83,4 mMol) (E)-4-(3-Methoxyphenyl)but-3-en-2-on und 7,67 g (83,4 mMol) Glyoxylsäure-monohydrat in 21,2 ml Essigsäure wird während 20 Stunden am Rückfluss gekocht. Das Reaktionsgemisch wird mit Wasser verdünnt und mit Essigester extrahiert. Die organische Phase wird zweimal mit 3N Natronlauge extrahiert; die vereinigten wässrigen Phasen werden bis zur stark sauren Reaktion mit 3N Salzsäure versetzt und zweimal mit Essigester extrahiert. Die vereinigten organischen Phasen werden über Magnesiumsulfat getrocknet und eingeengt. Kristallisation des Rückstandes aus Aether/Essigester liefert (E,E)-6-(3-Methoxyphenyl)-4-oxo-2,5-hexadiensäure vom Schmelzpunkt 158-160°.

Beispiel 7

Eine Lösung von 5 g (21 mMol) (E)-4-(3,4,5-Trimethoxyphenyl)but-3-en-2-on und 2 g (21,7 mMol) Glyoxylsäure-monohydrat in 5,2 ml Essigsäure wird während 20 Stunden am Rückfluss gekocht. Das Reaktionsgemisch wird mit Wasser verdünnt und mit Essigester extrahiert. Die organische Phase wird zweimal mit 3N Natronlauge extrahiert; die vereinigten wässrigen Phasen werden bis zur stark sauren Reaktion mit 3N Salzsäure versetzt und zweimal mit Essigester extrahiert. Die vereinigten organischen Phasen werden über Magnesiumsulfat getrocknet und eingeengt. Kristallisation des Rückstandes aus Methylenchlorid/Aether liefert (E,E)-4-Oxo-6-(3,4,5-trimethoxyphenyl)-2,5-hexadiensäure vom Schmelzpunkt 152-154°.

Beispiel 8

Eine Lösung von 15 g (78,8 mMol) (E)-4-[3,4-(Methylendioxy)phenyl]but-3-en-2-on und 7,2 g (78,8 mMol) Glyoxylsäure-monohydrat in 20 ml Essigsäure wird während 20 Stunden am Rückfluss gekocht. Das Reaktionsgemisch wird mit Wasser verdünnt und mit Essigester extrahiert. Die organische Phase wird zweimal mit 3N Natronlauge extrahiert; die vereinigten wässrigen Phasen werden bis zur stark sauren Reaktion mit 3N Salzsäure versetzt und zweimal mit Essigester extrahiert. Die vereinigten organischen Phasen werden über Magnesiumsulfat getrocknet und eingeengt. Kristallisation des Rückstandes aus Essigester/Aether liefert (E,E)-6-[3,4-(Methylendioxy)phenyl]-4-oxo-2,5-hexadiensäure vom Schmelzpunkt 170-173°.

Beispiel 9

Eine Lösung von 12 g (56 mMol) (E)-4-(4-Trifluormethylphenyl)but-3-en-2-on und 5,15 g (56 mMol) Glyoxylsäure-monohydrat in 14 ml Essigsäure wird während 20 Stunden am Rück fluss gekocht. Das Reaktionsgemisch wird mit Wasser verdünnt und mit Essigester extrahiert. Die organische Phase wird zweimal mit 3N Natronlauge extrahiert; die vereinigten wässrigen Phasen werden bis zur stark sauren Reaktion mit 3N Salzsäure versetzt und zweimal mit Essigester extrahiert. Die vereinigten organischen Phasen werden über Magnesiumsulfat getrocknet und eingeengt. Kristallisation des Rückstandes aus Aether/Hexan liefert (E,E)-4-Oxo-6-(4-trifluormethylphenyl)-2,5-hexadiensäure vom Schmelzpunkt 192-195°.

Beispiel 10

a) Eine Lösung von 16,6 g (0,1 Mol) 2,5-Dimethoxybenzaldehyd in 21 ml (0,28 Mol) Aceton und 10 ml Wasser wird mit 2,5 ml 3N Natronlauge versetzt, wobei die Temperatur des Reaktionsgemisches 30° nicht übersteigen soll. Man rührt das Gemisch 20 Stunden bei Raumtemperatur, verdünnt mit Wasser und setzt bis zur stark sauren Reaktion 3N Salzsäure zu. Die wässrige Phase wird zweimal mit Methylenchlorid extrahiert; die vereinigten organischen Phasen werden mit Wasser gewaschen, über Magnesiumsulfat getrocknet und eingeengt. Der Rückstand wird durch Flash-Chromatographie an 1000 g Silicagel (Elutionsmittel Methylenchlorid/Essigester 9:1) gereinigt. Man erhält (E)-4-(2,5-Dimethoxyphenyl)but-3-en-2-on als gelbes Oel.
MS: 206 ($M^+$) m/e.

b) Eine Lösung von 19 g (92 mMol) (E)-4-(2,5-Dimethoxyphenyl)but-3-en-2-on und 8,4 g (92 mMol) Glyoxylsäure-monohydrat in 23,4 ml Essigsäure wird während 20 Stunden am Rückfluss gekocht. Das Reaktionsgemisch wird mit Wasser verdünnt und mit Essigester extrahiert. Die organische Phase wird zweimal mit 3N Natronlauge extrahiert; die vereinigten wässrigen Phasen werden bis zur stark sauren Reaktion mit 3N Salzsäure versetzt und zweimal mit Essigester extrahiert. Die vereinigten organischen Phasen werden über Magnesiumsulfat getrocknet und eingeengt. Kristallisation des Rück standes aus Aether/Essigester liefert (E,E)-6-(2,5-Dimethoxyphenyl)-4-oxo-2,5-hexadiensäure vom Schmelzpunkt 145-146°.

Beispiel 11

a) Eine Lösung von 20 g (0,36 Mol) 2-Propin-1-ol in 637 ml (6,66 Mol) Aethylvinyläther wird bei 0° unter Argon mit 1,27 ml (16,7 mMol) Trifluoressigsäure versetzt, und anschliessend wird 65 Stunden bei Raumtemperatur gerührt. Man gibt 1,3 g Natriumcarbonat zu, rührt noch 30 Minuten bei Raumtemperatur und engt das Reaktionsgemisch am Rotationsverdampfer ein. Destillation des Rückstandes unter vermindertem Druck liefert 1-(1-Aethoxyäthoxy)-2-propin von Siedepunkt 55°/30 mmHg.
MS: 127 (M-H) m/e.

b) Eine Lösung von 34,74 g (168 mMol) Dicyclohexylcarbodiimid in 300 ml Methylenchlorid wird bei -10° mit einer Lösung von 10,3 g (168 mMol) N,O-Dimethylhydroxylamin in 100 ml Methylenchlorid versetzt. Man lässt das Gemisch noch 10 Minuten bei -10° rühren und gibt dann rasch eine Lösung von 30 g (168 mMol) 4-Methoxyzimtsäure in 100 ml Methylenchlorid zu. Das Gemisch wird noch 20 Stunden bei Raumtemperatur gerührt; dann wird mit 200 ml 3N Ammoniaklösung versetzt und zweimal mit je 200 ml Methylenchlorid extrahiert. Die vereinigten organischen Phasen werden über Magnesiumsulfat getrocknet

und eingeengt. Der Rückstand wird durch Flash-Chromatrographie an 1000 g Silicagel (Elutionsmittel Methylenchlorid/Essigester 9:1) gereinigt. Man erhält 4-Methoxyzimtsäure-N,O-dimethylhydroxamat als gelbes Oel.

MS: 221 ($M^+$), 161 (base peak) m/e.

c) Eine Lösung von 5,18 g (40 mMol) 1-(1-Aethoxyäthoxy-2-propin in 100 ml Tetrahydrofuran wird unter Argon bei -78° mit 25 ml n-Butyllithiumlösung (1,6M in Hexan) versetzt. Man lässt des Gemisch 30 Minuten bei -40° rühren, kühlt erneut auf -78° ab und gibt eine Lösung von 8,85 g (40 mMol) 4-Methoxyzimtsäure-N,O-dimethylhydroxamat in 100 ml Tetrahydrofuran zu. Das Reaktionsgemisch wird noch 10 Minuten bei -78° gerührt, dann auf 0° erwärmt und mit 100 ml gesättigter Ammoniumchlorid-Lösung versetzt. Die Wasserphase wird zweimal mit Aether extrahiert; die vereinigten organischen Phasen werden nacheinander mit gesättigter Kochsalzlösung und mit Wasser gewaschen, über Natriumsulfat getrocknet und eingeengt. Der Rückstand wird durch Flash-Chromatrographie an 500 g Silicagel (Elutionsmittel Methylenchlorid/Aether 4:1) gereinigt. Man erhält (E)-6-(1-Aethoxyäthoxy)-1-(4-methoxyphenyl)-1-hexen-4-in-3-on als rotes Oel.

MS: 288 ($M^+$), 45 (base peak) m/e.

IR (Film): 2217, 1629, 1599, 1512, 1249 $cm^{-1}$.

Beispiel 12

a) Eine Lösung von 20,6 (0,1 Mol) Dicyclohexylcarbodiimid in 250 ml Methylenchlorid wird bei -10° mit einer Lösung von 6,1 g (0,1 Mol) N,O-Dimethylhydroxylamin in 100 ml Methylenchlorid versetzt. Man lässt das Gemisch noch 10 Minuten bei -10° rühren und gibt dann rasch eine Lösung von 23 g (0,1 Mol) 3,4,5-Trimethoxyzimtsäure in 100 ml Methylenchlorid zu. Das Gemisch wird noch 20 Stunden bei Raumtemperatur gerührt; dann wird mit 200 ml 3N Ammoniaklösung versetzt und zweimal mit je 200 ml Methylenchlorid extrahiert. Die vereinigten organischen Phasen werden über Magnesiumsulfat getrocknet und eingeengt. Der Rückstand wird durch Flash-Chromatographie an 1000 g Silicagel (Elutionsmittel Methylenchlorid/Aether 4:1) gereinigt und anschliessend aus Aether kristallisiert. Man erhält 3,4,5-Trimethoxyzimtsäure-N,O-dimethylhydroxamat.

MS: 281 ($M^+$), 221 (base peak) m/e.

b) Eine Lösung von 5,18 g (40 mMol) 1-(1-Aethoxyäthoxy)-2-propin in 100 ml Tetrahydrofuran wird unter Argon bei -78° mit 25 ml n-Butyllithiumlösung (1,6M in Hexan) versetzt. Man lässt das Gemisch 30 Minuten bei -40° rühren, kühlt erneut auf -78° ab und gibt eine Lösung von 11,25 g (40 mMol) 3,4,5-Trimethoxyzimtsäure-N,O-dimethylhydroxamat in 100 ml Tetrahydrofuran zu. Das Reaktionsgemisch wird noch 10 Minuten bei -78° gerührt, dann auf 0° erwärmt und mit 100 ml gesättigter Ammoniumchlorid-Lösung versetzt. Die Wasserphase wird zweimal mit Aether extrahiert; die vereinigten organischen Phasen werden nacheinander mit gesättigter Kochsalzlösung und mit Wasser gewaschen, über Natriumsulfat getrocknet und eingeengt. Der Rückstand wird durch Flash-Chromatographie an 500 g Silicagel (Elutionsmittel Methylenchlorid/Aether 4:1) gereinigt. Man erhält (E)-6-(1-Aethoxyäthoxy)-1-(3,4,5-trimethoxyphenyl)-1-hexen -4-in-3-on als braunes Oel.

MS: 348 ($M^+$), 45 (base peak) m/e.

Beispiel 13

Eine Lösung von 3 g (10,4 mMol) (E)-6-(1-Aethoxyäthoxy)-1-(4-methoxyphenyl)-1-hexan-4-in-3-on in 50 ml Tetrahydrofuran wird bei Raumtemperatur mit 3 ml 1N Salzsäure versetzt und 30 Minuten bei Raumtemperatur gerührt. Man verdünnt das Reaktionsgemisch mit Wasser und extrahiert zweimal mit Aether. Die vereinigten organischen Phasen werden über Magnesiumsulfat getrocknet und eingeengt. Der Rückstand wird durch Flash-Chromatographie an 100 g Silicagel (Elutionsmittel Methylenchlorid/Aether 4:1) gereinigt und anschliessend aus Methylenchlorid/Aether/Hexan kristallisiert. Man erhält (E)-6-Hydroxy-1-(4-methoxypheny)-1-hexen-4-in-3-on vom Schmelzpunkt 68-69°.

Beispiel 14

Eine Lösung von 4 g (11,5 mMol) (E)-6-(1-Aethoxyäthoxy)-1-(3,4,5-trimethoxyphenyl)-1-hexan-4-in-3-on in 40 ml Aethanol/Tetrahydrofuran (1:1) wird bei Raumtemperatur mit 0,4 g (1,6 mMol) Pyridinium-(toluol-4-sulfonat) versetzt und 20 Stunden bei Raumtemperatur gerührt. Man verdünnt das Reaktionsgemisch mit Wasser und extrahiert zweimal mit Aether. Die vereinigten organischen Phasen werden über Magnesiumsulfat getrocknet und eingeengt. Der Rückstand wird durch Flash-Chromatographie an 200 g Silicagel (Elutionsmittel Methylenchlorid/Aether 4:1) gereinigt und anschliessend aus Methylenchlorid/Aether kristallisiert. Man erhält (E)-6-Hydroxy-1-(3,4,5-trimethoxypheny)-1-hexen-4-in-3-on vom Schmelzpunkt 113-115°.

Beispiel 15

a) Eine Lösung von 6,5 ml (0,11 Mol) 2-Propin-1-ol in 80 ml Tetrahydrofuran wird bei -78° unter Argon mit 13,5 g (0,12 Mol) Kalium-tert.-butylat versetzt. Man rührt 3 Stunden bei -78°, gibt dann 14,1 ml (0,12 Mol) 3,3-Dimethylallylbromid zu und rührt nochmals 3 Stunden bei -78° und 20 Stunden bei Raumtemperatur. Das Reaktionsgemisch wird auf Eis/Wasser gegossen und zweimal mit Aether extrahiert. Die vereinigten organischen Phasen werden über Magnesiumsulfat getrocknet und eingeengt. Man erhält 1-[(3-Methyl-2-butyenyl)oxy]-2-propin als gelbes Oel, welches ohne Reinigung weiterverwendet wird.
MS: 123 (M-H), 109 ($M-CH_3$) m/e.

b) Eine Lösung von 4,97 g (40 mMol) 1-[(3-Methyl-2-butenyl)oxy]-2-propin in 100 ml Tetrahydrofuran wird unter Argon bei -78° mit 25 ml einer n-Butyllithium-Lösung (1,6M in Hexan) versetzt. Man lässt das Gemisch 30 Minuten bei -40° rühren, kühlt erneut auf -78° ab und gibt dann eine Lösung von 8,85 g (40 mMol) 4-Methoxyzimtsäure-N,O-dimethylhydroxamat in 100 ml Tetrahydrofuran zu. Das Reaktionsgemisch wird noch 10 Minuten bei -78° gerührt, dann auf 0° erwärmt und mit 100 ml gesättigter Ammoniumchlorid-Lösung versetzt. Die Wasserphase wird zweimal mit Aether extrahiert; die vereinigten organischen Phasen werden nacheinander mit gesättigter Kochsalzlösung und mit Wasser gewaschen, über Magnesiumsulfat getrocknet und eingeengt. Der Rückstand wird durch Flash-Chromatographie an 700 g Silicagel (Elutionsmittel Methylenchlorid/Aether 4:1) gereinigt. Man erhält (E)-1-(4-Methoxyphenyl)-6-[(3-methyl-2-butenyl)oxy]-1-hexen -4-in-3-on als braunes Oel.
MS: 284 ($M^+$), 161 (base peak) m/e.
IR (Film): 2214, 1628, 1599, 1512, 1248, 1173, 1078, 1028 $cm^{-1}$.

Beispiel 16

a) Eine Lösung von 41,26 g (0,2 Mol) Dicyclohexylcarbodiimid in 250 ml Methylenchlorid wird bei -10° mit einer Lösung von 12,2 g (0,2 Mol) N,O-Dimethylhydroxylamin in 250 ml Methylenchlorid versetzt. Man lässt das Gemisch noch 10 Minuten bei -10° rühren und gibt dann rasch eine Lösung von 38,8 g (0,2 Mol) 4-Hydroxy-3-methoxyzimtsäure in 250 ml Methylenchlorid zu. Das Gemisch wird noch 20 Stunden bei Raumtemperatur gerührt, mit 500 ml 3N Ammoniaklösung versetzt und zweimal mit Methylenchlorid extrahiert. Die vereinigten organischen Phasen werden über Magnesiumsulfat getrocknet und eingeengt. Der Rückstand wird durch Flash-Chromatographie an 1000 g Silicagel (Elutionsmittel Methylenchlorid/Aether 4:1) gereinigt und anschliessend aus Methylenchlorid/Aether/Hexan kristallisiert. Man erhält 4-Hydroxy-3-methoxyzimtsäure-N,O-dimethylhydroxamat vom Schmelzpunkt 85-86°.

b) Eine Lösung von 7,6 g (61,7 mMol) 1-[(3-Methyl-2-butenyl)oxy]-2-propin in 100 ml Tetrahydrofuran wird unter Argon bei -78° mit 38,5 ml n-Butyllithiumlösung (1,6M in Hexan) versetzt. Man lässt das Gemisch 30 Minuten bei -40° rühren, kühlt erneut auf -78° ab und gibt eine Lösung von 7,3 g (30,8 mMol) 4-Hydroxy-3-methoxyzimtsäure-N,O-dimethylhydroxamat in 50 ml Tetrahydrofuran zu. Das Reaktionsgemisch wird noch 30 Minuten bei -78° gerührt, dann auf 0° erwärmt und mit 100 ml gesättigter Ammoniumchlorid-Lösung versetzt. Die Wasserphase wird zweimal mit Aether extrahiert; die vereinigten organischen Phasen werden nacheinander mit gesättigter Kochsalzlösung und mit Wasser gewaschen, über Magnesiumsulfat getrocknet und eingeengt. Der Rückstand wird durch Flash-Chromatographie an 500 g Silicagel (Elutionsmittel Methylenchlorid/Aether 4:1) gereinigt. Man erhält (E)-1-(4-Hydroxy-3-methoxyphenyl)-6-[(3-methyl-2-butenyl)oxy]-1-hexen-4-in-3-on als rotes Oel.
MS: 300 ($M^+$), 177 (base peak) m/e.
IR (Film): 3367, 2213, 1625, 1567, 1513 $cm^{-1}$.

Beispiel 17

a) Eine Lösung von 5 ml (60 mMol) Propiolsäuremethylester in 100 ml Tetrahydrofuran wird unter Argon bei -78° mit 41,3 ml n-Butyllithium-Lösung (1,6M in Hexan) versetzt. Man rührt das Gemisch 10 Minuten bei -78° und gibt dann eine Lösung von 9,73 g (60 mMol) 4-Methoxyzimtaldehyd in 100 ml Tetrahydrofuran zu. Das Reaktionsgemisch wird 20 Minuten bei -78° gerührt, dann auf -20° erwärmt und hierauf mit 100 ml gesättigter Ammoniumchloridlösung versetzt. Die Wasserphase wird zweimal mit Aether extrahiert; die vereinigten organischen Phasen werden mit Wasser gewaschen, über Natriumsulfat getrocknet und eingeengt. Flash-Chromatographie des Rückstandes an 1000 g Silicagel (Elutionsmittel Methylenchlorid/Essigester 9:1) liefert (E)-4-Hydroxy-6-(4-methoxyphenyl)-5-hexen -2-insäuremethylester als gelbes Oel.

MS: 246 ($M^+$), 121 (base peak) m/e.

IR (Film): 3400, 2950, 2236, 1716, 1606, 1518, 1435, 1252, 1031 $cm^{-1}$.

b) Eine Lösung von 10,7 g (43,4 mMol) (E)-4-Hydroxy-6-(4-methoxyphenyl)-5-hexen -2-insäuremethylester in 150 ml Methylenchlorid wird bei 0° zu einer Suspension von 56,7 g (652 mMol) Mangandioxid in 200 ml Methylenchlorid getropft. Das Reaktionsgemisch wird 2 Stunden bei 0° gerührt, über Magnesiumsulfat filtriert und eingeengt. Kristallisation des Rückstandes aus Aether liefert (E)-6-(4-Methoxyphenyl)-4 -oxo-5-hexen-2-insäuremethylester vom Schmelzpunkt 70°.

Beispiel 18

Eine Lösung von 5,3 g (21,7 mMol) (E)-6-(4-Methoxyphenyl)-4-oxo-5-hexen-2-insäuremethylester in 70 ml Tetrahydrofuran wird bei 0° innerhalb von 15 Minuten mit 48,6 ml (26 mMol) einer 3%igen wässrigen Kaliumhydroxid-Lösung versetzt. Das Reaktionsgemisch wird 10 Minuten bei 0° gerührt, dann mit 100 ml Wasser verdünnt und einmal mit 100 ml Aether extrahiert. Die organische Phase wird verworfen. Die wässrige Phase wird durch vorsichtige Zugabe von 1N Salzsäure auf pH 1 gestellt und zweimal mit Aether extrahiert. Die vereinigten organischen Phasen werden mit Wasser gewaschen, über Natriumsulfat getrocknet und eingeengt. Kristallisation des Rückstandes aus Aether/Hexan liefert (E)-6-(4-Methoxyphenyl)-4-oxo-5-hexen-2-insäure vom Schmelzpunkt 98-100°.

Beispiel 19

Eine Lösung von 20 g (8,12 mMol) (E)-4-Hydroxy-6-(4-methoxyphenyl)-5-hexen-2-insäuremethylester und 62 mg (~5 Mol%) 2,8-Diazabicyclo[5.4.0]undec-7-en in 80 ml Methylenchlorid wird während 35 Stunden unter Argon bei 0° gerührt. Danach gibt man 25 ml 0,1N Salzsäure zu und trennt die Phasen. Die wässrige Phase wird zweimal mit 25 ml Methylenchlorid extrahiert; die vereinigten organischen Fraktionen werden über Magnesiumsulfat getrocknet, filtriert und eingedampft. Der Rückstand wird an 130 g Kieselgel mit Petroläther/Aether (1:1) chromatographiert, wobei zuerst (E,E)-6-(4-Methoxyphenyl)-4-oxo-2,5-hexadiensäuremethylester vom Schmelzpunkt 103-103,5° und dann (2Z,5E)-6-(4-Methoxyphenyl)-4-oxo-2,5-hexadiensäuremethylester vom Schmelzpunkt 54-55° erhalten werden.

Beispiel 20

Eine Lösung von 1,70 g (6,9 mMol) (2Z,5E)-6-(4-Methoxyphenyl)-4-oxo-2,5-hexadiensäuremethylester in 20 ml Aether wird unter Eiskühlung und heftigem Rühren innerhalb von ca. 15 Minuten mit 20 ml 5%iger Kaliumhydroxidlösung versetzt. Das Reaktionsgemisch wird während 1 Stunde bei 4° gerührt und dann mit 15 ml Phosphatpuffer (pH = 6,5) und 30 ml Essigester versetzt, worauf die Phasen getrennt werden. Die wässrige Phase wird zweimal mit Essigester extrahiert; die vereinigten organischen Phasen werden über Magnesiumsulfat getrocknet, filtriert und eingedampft. Der feste Rückstand wird aus Essigester/Hexan umkristallisiert, wobei man (2Z,5E)-6-(4-Methoxyphenyl)-4-oxo-2,5-hexadiensäure als orangefarbiges Pulver vom Schmelzpunkt 103,5-104° erhält.

Beispiel 21

a) 12,3 ml (0,2 Mol) Propiolsäure werden zusammen mit 23,6 ml (0,2 Mol) Diäthylenglykolmonomethyläther, 1,5 g (8 mMol) p-Toluolsulfonsäuremonohydrat und 80 ml Toluol während 20 Stunden am Rückfluss gekocht, wobei das entstehende Reaktionswasser azeotrop abdestilliert und in einem Wasserabscheider gesammelt wird. Nach beendeter Wasserabscheidung wird das Reaktionsgemisch nacheinander mit gesättigter Natriumhydrogencarbonat-Lösung und mit Wasser gewaschen; die Toluolphase wird über Natriumsulfat getrocknet und eingeengt. Das Rohprodukt wird durch Flash-Chromatographie an 500 g Silicagel (Elutionsmittel Methylenchlorid/Essigester 9:1) gereinigt. Man erhält Propiolsäure-[2-(2-methoxyäthoxy)äthyl]ester als farbloses Oel.

b) Eine Lösung von 1,75 g (10,2 mMol) Propiolsäure-[2-(2-methoxyäthoxy)äthyl]ester in 20 ml Tetrahydrofuran wird unter Argon bei -78° mit 7 ml n-Butyllithium-Lösung (1,6M in Hexan) versetzt. Man rührt das Gemisch 5 Minuten bei -78° und gibt dann innerhalb von 10 Minuten eine Lösung von 1,65 g (10,2 mMol) 4-Methoxyzimtaldehyd in 25 ml Tetrahydrofuran zu. Das Reaktionsgemisch wird 5 Minuten bei -78° gerührt und dann mit 50 ml gesättigter Ammoniumchlorid-Lösung versetzt. Die wässrige Phase wird zweimal mit Aether extrahiert; die vereinigten organischen Phasen werden mit Wasser gewaschen, über Natriumsulfat getrocknet, filtriert und eingeengt. Flash-Chromatographie des Rückstandes an 120 g Silicagel (Elutionsmittel Methylenchlorid/Aether 4:1) liefert (E)-4-Hydroxy-6-(4-methoxyphenyl)-5-hexen-2-insäure-[2-(2-methoxyäthoxy)äthyl]ester als gelbes Oel.
MS: 334 ($M^+$), 214, 186, 158, 59, 45 (base peak) m/e.
IR (Film): 3395, 2236, 1713, 1606, 1512, 1252 $cm^{-1}$.

c) Eine Lösung von 2,4 g (7,2 mMol) (E)-4-Hydroxy-6-(4-methoxyphenyl)-5-hexen-2-insäure -[2-(2-methoxyäthoxy)äthyl]ester in 40 ml Methylenchlorid wird bei 0° zu einer Suspension von 9,4 g (108 mMol) Mangandioxid in 30 ml Methylenchlorid getropft. Das Reaktionsgemisch wird 2 Stunden bie 0° gerührt, über Magnesiumsulfat filtriert und eingeengt. Kristallisation des Rückstandes aus Aether/Hexan liefert (E)-6-(4-Methoxyphenyl)-4-oxo-5-hexen-2-insäure-[2-(2-methoxyäthoxy)äthyl]ester vom Schmelzpunkt 41-42°.

Beispiel 22

a) Eine Lösung von 13,9 g (0,1 Mol) Bromessigsäure in 40 ml Toluol wird mit 11,8 ml (0,1 Mol) Diäthylenglykolmonomethyläther und 0,8 g p-Toluolsulfonsäure-monohydrat versetzt. Das Gemisch wird am Wasserabscheider unter Rückfluss erhitzt, bis sich kein Wasser mehr abscheidet. Das Reaktionsgemisch wird nacheinander mit Wasser, gesättigter Natriumhydrogencarbonat-Lösung und Wasser gewaschen. Die organische Phase wird über Natriumsulfat getrocknet und eingeengt. Flash-Chromatographie des Rückstandes an 500 g Silicagel (Elutionsmittel Methylenchlorid/Aether 4:1) liefert Bromessigsäure-[2-(2-methoxyäthoxy)äthyl]ester als hellgelbes Oel.
IR (Film): 1740, 1285, 1110 $cm^{-1}$.

b) Eine Lösung von 12,8 g (49 mMol) Triphenylphosphin in 80 ml Toluol wird innerhalb von 10 Minuten mit einer Lösung von 10,7 g (44,3 mMol) Bromessigsäure-[2-(2-methoxyäthoxy)äthyl]ester in 20 ml Toluol versetzt. Das Reaktionsgemisch wird während 4 Stunden bei Raumtemperatur gerührt. Der Niederschlag wird abfiltriert, mit Toluol gewaschen und im Wasserstrahlvakuum getrocknet. Man erhält [[[2-(2-Methoxyäthoxy)äthoxy] carbonyl]methyl]triphenylphosphoniumbromid, welches direkt weiterverarbeitet wird.

c) Eine Lösung von 21 g (41,7 mMol) [[[2-(2-Methoxyäthoxy)äthoxy] carbonyl]methyl]-triphenylphosphoniumbromid in 600 ml Wasser wird bis zur alkalischen Reaktion tropfenweise mit ca. 50 ml 1N Natriumhydroxid-Lösung versetzt. Das Reaktionsgemisch wird mit 500 ml Methylenchlorid extrahiert; die organische Phase wird über Natriumsulfat getrocknet und eingeengt. Man erhält [[[2-(2-Methoxyäthoxy)-äthoxy]carbonyl]methylen]triphenylphosphoran.
MS: 422 ($M^+$), 301 (base peak) m/e.

d) Eine Lösung von 14,3 g (50 mMol) 5,5-Dibrombarbitursäure in 300 ml Aether wird mit 17,6 (0,1 Mol) 4-(3-Methoxyphenyl)-3-buten-2-on versetzt, und das Gemisch wird während 20 Stunden bei Raumtemperatur gerührt. Die ausgefallene Barbitursäure wird abfiltriert; das Filtrat wird nacheinander mit gesättigter Natriumhydrogencarbonat-Lösung und mit Wasser gewaschen. Die organische Phase wird über Natriumsulfat getrocknet und eingeengt. Flash-Chromatographie des Rückstandes an 1000 g Silicagel (Elutionsmittel Methylenchlorid/Essigester 9:1) liefert (E)-1-Brom-4-(3-methoxyphenyl)-3-buten-2-on als gelbes Oel.
MS: 256, 254 ($M^+$), 161 (base peak) m/e.
IR (Film): 1697, 1611, 1577, 1263, 990 $cm^{-1}$.

e) Eine Lösung von 14,9 g (35,2 mMol) [[[2-(2-Methoxyäthoxy)äthoxy]carbonyl] methylen]-triphenylphosphoran (Herstellung siehe Absatz c)) in 100 ml Toluol wird mit 4,48 g (17,6 mMol) (E)-1-Brom-4-(3-methoxyphenyl)-3-buten-2-on (Herstellung siehe Absatz d)) versetzt. Das Reaktionsgemisch wird während 2 Stunden am Rückfluss gerührt und dann filtriert; das Filtrat wird nach Zugabe von 1,63 ml (17,6 mMol) Bromessigsäureähylester nochmals während 2 Stunden am Rückfluss gerührt. Der ausgefallene Festkörper wird abfiltriert; das Filtrat wird eingeengt und durch Flash-Chromatographie an 500 g Silicagel (Elutionsmittel Aether/Hexan 10:1) gereinigt. Kristallisation des gereinigten Produktes aus Methylenchlorid/Aether/Hexan liefert (E,E)-6-(3-Methoxyphenyl)-4-oxo-2,5-hexadiensäure -[2-(2-methoxyäthoxy)äthyl]ester vom Schmelzpunkt 59-60°.
MS: 334 ($M^+$), 215, 187 (base peak) m/e.

Beispiel 23

a) Zu einer Lösung von 5,3 ml (37,0 mMol) 3,3-Diäthoxy-1-propin in 100 ml absolutem Tetrahydrofuran werden bei -78° 23,1 ml n-Butyllithium-Lösung (1,6M in Hexan) zugegeben, worauf noch 30 Minuten bei -78° gerührt wird. Danach wird eine Lösung von 5,0 g (30,83 mMol) 4-Methoxyzimtaldehyd in 25 ml Tetrahydrofuran langsam zugetropft. Das Reaktionsgemisch wird während 2 Stunden bei -78° gerührt und anschliessend bei -40° mit 50 ml gesättigter Ammoniumchloridlösung, 50 g Eis und 150 ml Aether versetzt. Die wässrige Phase wird zweimal mit Aether extrahiert; die vereinigten organischen Fraktionen werden über Magnesiumsulfat getrocknet, filtriert und eingedampft. Der ölige Rückstand wird an 300 g Kieselgel mit Aether/Petroläther (1:1) chromatographiert, worauf man (E)-6,6-Diäthoxy-1-(p-methoxyphenyl)-1-hexen-4-in-3-ol als leicht gelbliches Oel erhält, welches beim längeren Stehenlassen langsam erstarrt.
IR (Film): 3414(m), 2976(m), 2892(w), 2240(w), 1652(w), 1607(s), 1512(s), 1328(m), 1252(s), 1142(s), 1051-(s), 825(m).

b) Eine Lösung von 3,0 g (10,33 mMol) (E)-6,6-Diäthoxy-1-(p-methoxyphenyl)-1-hexen-4-in-3-ol in 15 ml Methylenchlorid wird bei 0° zu einer Suspension von 27 g Mangandioxid in 45 ml Methylenchlorid gegeben. Das Reaktionsgemisch wird anschliessend noch 1 Stunde bei Raumtemperatur gerührt, über Magnesiumsulfat filtriert und eingedampft. Der Rückstand wird an 100 g Kieselgel mit Petroläther/Aether (2:1) chromatographiert, worauf man (E)-6,6-Diäthoxy-1-(p-methoxyphenyl)-1-hexen-4-in-3-on als leicht gelbliches Oel erhält.
IR (Film): 2977(m), 2934(w), 2220(w), 1630(s), 1599(s), 1512(s), 1423(m), 1246(s), 1174(s), 1055(s), 830(m).

Beispiel 24

Eine Lösung von 1,0 g (3,47 mMol) (E)-6,6-Diäthoxy-1-(p-methoxyphenyl)-1-hexen-4-in-3-on in 4 ml Dioxan und 2 ml 2,2N wässriger Bromwasserstoffsäure wird während 18 Stunden bei 30° gerührt und anschliessend mit 20 ml Aether und 10 ml Wasser versetzt. Die wässrige Phase wird zweimal mit Essigester extrahiert; die vereinigten organischen Fraktionen werden über Magnesiumsulfat getrocknet, filtriert und eingedampft. Der Rückstand wird an 80 g Kieselgel mit Acetonitril/Wasser (1:1) chromatographiert. Die das Produkt enthaltenden Fraktionen werden mit Essigester extrahiert; die vereinigten Essigesterextrakte werden über Magnesiumsulfat getrocknet, filtriert und eingedampft. Der Rückstand wird aus Essigester/Hexan umkristallisiert, wobei man (E,E)-6-(p-Methoxyphenyl)-4-oxo-2,5-hexadiensäure vom Schmelzpunkt 138-139° erhält.

Beispiel A

Kristalline Verbindungen der Formeln I und II und pharmazeutisch verwendbare Salze von Verbindungen der Formel I können als Wirkstoffe zur Herstellung von Hartgelatinekapseln verwendet werden, deren Inhalt pro Kapsel folgende Zusammensetzung aufweist:

| | |
|---|---|
| Wirkstoff | 50-250,0 mg |
| Milchzucker pulv. | 40,0 mg |
| Milchzucker krist. | 230- 30,0 mg |
| Maisstärke weiss | 20,0 mg |
| Talk | 8,0 mg |
| Magnesiumstearat | 2,0 mg |
| Füllgewicht pro Kapsel | 250,0 mg |

Der Wirkstoff und die Hilfsstoffe werden miteinander vermischt, und das Gemisch wird in Hartgelatinekapseln geeigneter Grösse abgefüllt. Erforderlichenfalls werden die Kapseln anschliessend mit einem magensaftresistenten Lack, bestehend aus Hydroxypropylmethylcellulosephthalat, versehen.

Beispiel B

Nicht kristalline Verbindungen der Formeln I und II können wie nachstehend beschrieben als Wirkstoffe zur Herstellung von Weichgelatinekapseln verwendet werden; die verwendeten Abkürzungen haben dabei folgende Bedeutung:

BHA = Butyliertes Hydroxyanisol
BHT = Butyliertes Hydroxytoluol
PEG = Polyäthylenglykol

a) 0,2 mg BHA und 1,0 mg Ascorbylpalmitat werden in 400 mg PEG 400 bei Raumtemperatur unter Stickstoffatmosphäre gelöst. Die Lösung wird mit 50-250 mg Wirkstoff bei Raumtemperatur unter Stickstoff versetzt. Nachdem alles gelöst ist, wird das erhaltene Gemisch in flüssiger Form in Weichgelatinekapseln abgefüllt.

b) 300 mg PEG 400 und 100 mg PEG 4000 werden unter Stickstoff erwärmt, bis sich das Gemisch verflüssigt hat. Danach werden unter Stickstoff 0,1 mg BHA, 0,1 mg BHT und 1,0 mg Ascorbylpalmitat zugesetzt. Nachdem sich alles gelöst hat, werden 50-250 mg Wirkstoff unter Stickstoff zugefügt und unter Durchmischung gelöst. Die Flüssigkeit wird dann in Weichgelatinekapseln abgefüllt.

c) 0,2 mg BHA, 0,2 mg BHT und 1,0 mg Ascorbylpalmitat werden in 400 mg Polysorbat-80 bei Raumtemperatur unter Stickstoff gelöst. Das Gemisch wird unter Stickstoff mit 50-250 mg Wirkstoff versetzt. Nachdem sich alles gelöst hat, wird die Flüssigkeit in Weichgelatinekapseln abgefüllt.

d) Ein Gemisch von je 200 mg Polysorbat-60 und Polysorbat-80 wird erwärmt. Das erhaltene flüssige Gemisch wird unter Stickstoff mit 0,2 mg BHA, 1,0 mg α-Tocopherol und 2,0 mg Ascorbylpalmitat versetzt. Nachdem alles gelöst ist, werden 50-250 mg Wirkstoff unter Stickstoff zugesetzt. Nach Durchmischung bis zur vollständigen Lösung wird das erhaltene Gemisch in Weichgelatinekapseln abgefüllt.

**Ansprüche**

1. Styrylketone der allgemeinen Formel

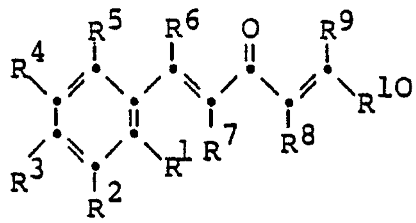

I

worin

$R^1$, $R^2$, $R^3$, $R^4$ und $R^5$ je Wasserstoff, Halogen, niederes Alkyl, niederes Alkoxy-niederes-alkyl, Hydroxy, niederes Alkoxy, niederes Alkenyloxy, niederes Alkinyloxy, niederes Alkoxy-niederes-alkoxy, Acyloxy, Aryl-niederes-alkoxy, niederes Alkylthio, niederes Alkoxy-niederes-alkylthio, niederes Alkenylthio, niederes Alkinylthio, Aryl-niederes-alkylthio, gegebenenfalls substituiertes Amino oder Trifluormethyl oder zwei benachbarte dieser Substituenten gemeinsam und zusammen mit den Kohlenstoffatomen, an welche sie gebunden sind, einen 5-bis 7-gliedrigen Ring bedeuten, wobei von den Substituenten $R^1$ bis $R^5$ mindestens zwei Wasserstoff bedeuten und mindestens einer von Wasserstoff verschieden ist;
$R^6$ und $R^7$ je Wasserstoff oder niederes Alkyl bedeuten;
$R^8$ und $R^9$ je Wasserstoff oder niederes Alkyl oder zusammen eine zusätzliche Kohlenstoff-Kohlenstoff-Bindung bedeuten;
einen Rest der Formel

$-COOR^{11}$, (a) $-CONR^{12}R^{13}$, (b) $-C(R^{14})=O$ (c)

$-C(R^{15})(OR^{16})_2$, (d) $-C(OR^{17})_3$ (e) oder

$-C(R^{18})(R^{19})OR^{20}$ (f)

bedeutet;
$R^{11}$ Wasserstoff, niederes Alkyl, niederes Alkenyl, niederes Alkoxy-niederes-alkyl, niederes Alkoxy-niederes-alkoxy-niederes-alkyl, Aryl oder Aryl-niederes-alkyl bedeutet;
$R^{12}$ und $R^{13}$ je Wasserstoff oder niederes Alkyl oder gemeinsam und zusammen mit dem Stickstoffatom einen 5-bis 7-gliedrigen gesättigten heterocyclischen Rest bedeuten;
$R^{14}$ niederes Alkyl, Aryl oder Aryl-niederes-alkyl bedeutet;
$R^{15}$ Wasserstoff, niederes Alkyl, Aryl oder Aryl-niederes-alkyl bedeutet;
$R^{16}$ niederes Alkyl oder niederes Alkoxy-niederes-alkyl bedeutet;
$R^{17}$ niederes Alkyl bedeutet;
$R^{18}$ und $R^{19}$ je Wasserstoff, niederes Alkyl, Aryl oder Aryl-niederes-alkyl bedeuten; und
$R^{20}$ Wasserstoff, niederes Alkyl, niederes Alkoxy-niederes-alkyl, niederes Alkenyl, niederes Alkinyl, Acyl oder Aryl-niederes-alkyl bedeuten;
wobei die im Molekül vorkommende(n) Doppelbindung(en) die E-und/oder Z-Konfiguration aufweist (aufweisen); sowie pharmazeutisch verwendbare Salze von sauren Verbindungen der Formel I mit Basen und von basischen Verbindungen der Formel I mit Säuren.

2. Verbindungen gemäss Anspruch 1, worin $R^1$ und $R^2$ je Wasserstoff und $R^3$, $R^4$ und $R^5$ je niederes Alkoxy oder $R^1$ und $R^5$ je Wasserstoff und $R^2$, $R^3$ und $R^4$ je niederes Alkoxy oder $R^1$, $R^2$ und $R^4$ je Wasserstoff und $R^3$ und $R^5$ je niederes Alkoxy oder $R^1$, $R^3$ und $R^4$ je Wasserstoff und $R^2$ und $R^5$ je niederes Alkoxy oder $R^1$, $R^2$ und $R^5$ je Wasserstoff und $R^3$ Hydroxy und $R^4$ niederes Alkoxy oder $R^3$ und $R^4$ zusammen niederes Alkylendioxy oder $R^1$, $R^2$, $R^4$ und $R^5$ je Wasserstoff und $R^3$ Halogen, niederes Alkoxy, niederes Alkylthio oder Trifluormethyl oder $R^1$, $R^2$, $R^3$ und $R^5$ je Wasserstoff und $R^4$ niederes Alkoxy bedeuten.

3. Verbindungen gemäss Anspruch 2, worin $R^1$, $R^2$, $R^4$ und $R^5$ je Wasserstoff und $R^3$ Fluor oder Methoxy oder $R^1$, $R^2$, $R^3$ und $R^5$ je Wasserstoff und $R^4$ Methoxy bedeuten.

4. Verbindungen gemäss einem der Ansprüche 1 bis 3, worin $R^6$ und $R^7$ je Wasserstoff bedeuten.

5. Verbindungen gemäss einem der Ansprüche 1 bis 4, worin $R^8$ und $R^9$ je Wasserstoff oder zusammen eine zusätzliche Kohlenstoff-Kohlenstoff-Bindung bedeuten.

6. Verbindungen gemäss einem der Ansprüche 1 bis 5, worin $R^{10}$ einen Rest der in Anspruch 1 definierten Formel (a), (d) oder (f), $R^{11}$ Wasserstoff, niederes Alkyl oder niederes Alkoxy-niederes-alkoxy-niederes-alkyl, $R^{15}$ Wasserstoff, $R^{16}$ niederes Alkyl, $R^{18}$ und $R^{19}$ je Wasserstoff und $R^{20}$ Wasserstoff, niederes Alkoxy-niederes-alkyl oder niederes Alkenyl bedeuten.

7. Verbindungen gemäss Anspruch 6, worin $R^{10}$ einen Rest der in Anspruch 1 definierten Formel (a) oder (f), $R^{11}$ Wasserstoff, Methyl oder Methoxyäthoxyäthyl und $R^{20}$ Wasserstoff oder 1-Aethoxyäthyl bedeuten.

8. (E,E)-6-(3-Methoxyphenyl)-4-oxo-2,5-hexadiensäure.

9. (E,E)-6-(4-Methoxyphenyl)-4-oxo-2,5-hexadiensäure.

10. (2Z,5E)-6-(4-Methoxyphenyl)-4-oxo-2,5-hexadiensäuremethylester.

11. (E)-6-(4-Methoxyphenyl)-4-oxo-5-hexen-2-insäure.

12. (E,E)-6-(4-Fluorphenyl)-4-oxo-2,5-hexadiensäure;
(2Z,5E)-6-(4-Methoxyphenyl)-4-oxo-2,5-hexadiensäure;
(E,E)-6-(3-Methoxyphenyl)-4-oxo-2,5-hexadiensäure-[2-(2-methoxyäthoxy)äthyl]ester; und
(E)-6-(4-Methoxyphenyl)-4-oxo-5-hexen-2-insäure-[2-(2-methoxyäthoxy)äthyl]ester.

13. Verbindungen allgemeinen Formel

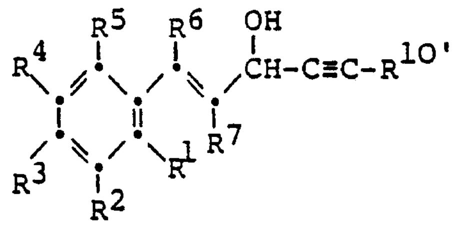

II

worin $R^1$, $R^2$, $R^3$, $R^4$, $R^5$, $R^6$ und $R^7$ die in Anspruch 1 angegebene Bedeutung besitzen und $R^{10'}$ einen Rest der in Anspruch 1 definierten Formel (a), (b), (d) oder (e) oder einen Rest der Formel

$$-C(R^{18})(R^{19})OR^{20'}$$ bedeutet,

(f')

worin $R^{18}$ und $R^{19}$ die in Anspruch 1 angegebene Bedeutung besitzen und $R^{20'}$ die in Anspruch 1 für $R^{20}$ definierte Bedeutung besitzt, jedoch nich Wasserstoff bedeutet, wenn $R^{18}$ und/oder $R^{19}$ Wasserstoff bedeutet.

14. Verbindungen gemäss Anspruch 13, worin $R^{10'}$ einen Rest der in Anspruch 1 definierten Formel (a) bedeutet.

15. Verbindungen gemäss Anspruch 14, worin $R^{11}$ niederes Alkyl oder niederes Alkoxy-niederes-alkoxy-niederes-alkyl bedeutet.

16. (E)-4-Hydroxy-6-(4-methoxyphenyl)-5-hexen-2-insäuremethylester.

17. (E)-4-Hydroxy-6-(4-methoxyphenyl)-5-hexen-2-insäure[2-(2-methoxyäthoxy)äthyl]ester.

18. Verbindungen gemäss einem der Ansprüche 1 bis 17 zur Anwendung als therapeutische Wirkstoffe, insbesondere als mucosaprotektive und/oder magensäuresekretionshemmende Wirkstoffe.

19. Verfahren zur Herstellung von Verbindungen gemäss einem der Ansprüche 1 bis 12, dadurch gekennzeichnet, dass man

a) eine Verbindung der allgemeinen Formel

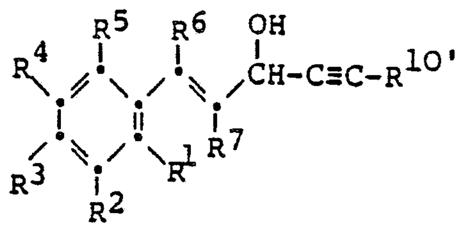

II

worin $R^1$, $R^2$, $R^3$, $R^4$, $R^5$, $R^6$ und $R^7$ die in Anspruch 1 angegebene Bedeutung besitzen und $R^{10'}$ einen Rest der in Anspruch 1 definierten Formel (a), (b), (d) oder (e) oder einen Rest der Formel

$$-C(R^{18})(R^{19})OR^{20'}$$

(f')

bedeutet,

worin $R^{18}$ und $R^{19}$ die in Anspruch 1 angegebene Bedeutung besitzen und $R^{20'}$ die in Anspruch 1 für $R^{20}$ definierte Bedeutung besitzt, jedoch nicht Wasserstoff bedeutet, wenn $R^{18}$ und/oder $R^{19}$ Wasserstoff bedeutet,

oxydiert; oder

b) eine Verbindung der obigen Formel II, worin $R^{10'}$ einen Rest der in Anspruch 1 definierten Formel (a) bedeutet, wobei aber $R^{11}$ nicht Wasserstoff bedeutet, mit einer Base behandelt; oder

c) eine Verbindung der allgemeinen Formel

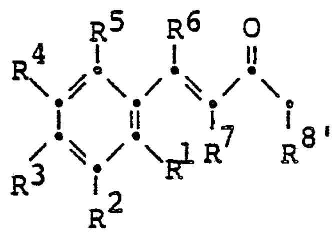

III

worin $R^1$, $R^2$, $R^3$, $R^4$, $R^5$, $R^6$ und $R^7$ die in Anspruch 1 angegebene Bedeutung besitzen und $R^{8'}$ Wasserstoff oder niederes Alkyl bedeutet,

mit einer Verbindung der allgemeinen Formel

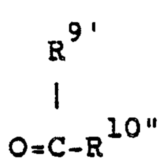

IV

worin $R^{9'}$ Wasserstoff oder niederes Alkyl und $R^{10''}$ einen Rest der in Anspruch 1 definierten Formel (a) bedeuten, wobei $R^{11}$ Wasserstoff bedeutet,

umsetzt; oder

d) eine Verbindung der allgemeinen Formel

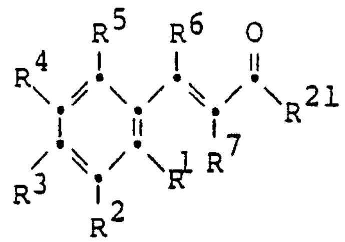

V

worin $R^1$, $R^2$, $R^3$, $R^4$, $R^5$, $R^6$ und $R^7$ die in Anspruch 1 angegebene Bedeutung besitzen und $R^{21}$ eine Abgangsgruppe bedeutet,
mit einer Verbindung der allgemeinen Formel

$H{\equiv}C\text{-}R^{10'''}$ VI

worin $R^{10'''}$ einen Rest der in Anspruch 1 definierten Formel (a), (b), (d), (e) oder (f) bedeutet,
umsetzt; oder

e) eine Verbindung der allgemeinen Formel

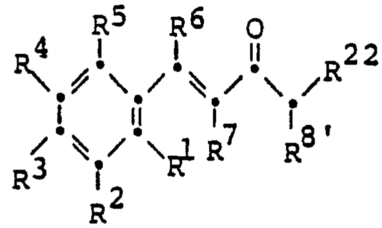

VII

worin $R^1$, $R^2$, $R^3$, $R^4$, $R^5$, $R^6$ und $R^7$ die in Anspruch 1 angegebene und $R^{8'}$ obige Bedeutung besitzen und $R^{22}$ Halogen bedeutet,
mit einer Verbindung der allgemeinen Formel

$(R^{23})_3P = CH\text{-}R^{10iv}$ VIII

worin $R^{23}$ einen Arylrest und $R^{10iv}$ einen Rest der in Anspruch 1 definierten Formel (a), (b), (d), (e) oder (f) bedeutet, wobei aber $R^{11}$ und $R^{20}$ nicht Wasserstoff bedeuten,
umsetzt; oder

f) von einer Verbindung der allgemeinen Formel

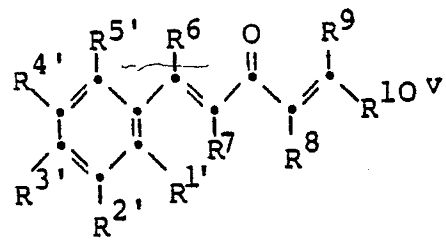

IX

worin $R^{1'}$, $R^{2'}$, $R^{3'}$, $R^{4'}$ und $R^{5'}$ die in Anspruch 1 für $R^1$, $R^2$, $R^3$, $R^4$ und $R^5$ angegebene Bedeutung besitzen und maximal drei davon zusätzlich geschütztes Hydroxy, geschütztes Amino oder geschütztes niederes Alkylamino bedeuten können, $R^6$, $R^7$, $R^8$ und $R^9$ die in Anspruch 1 angegebene Bedeutung besitzen, $R^{10v}$ einen Rest der in Anspruch 1 definierten Formel (a), (b), (c), (d) oder (e) oder der Formel

$$-C(R^{18})(R^{19})OR^{20''}$$

(f")

worin $R^{18}$ und $R^{19}$ die in Anspruch 1 angegebene Bedeutung besitzen und $R^{20''}$ die in Anspruch 1 für

$R^{20}$ angegebene Bedeutung besitzt und zusätzlich eine Schutzgruppe bedeuten kann, wobei das Molekül mindestens eine Schutzgruppe enthält,
die Schutzgruppe(n) abspaltet; oder

g) eine Verbindung der Formel I, woring $R^{10}$ einen Rest der in Anspruch 1 definierten Formel (a) bedeutet, wobei $R^{11}$ von Wasserstoff verschieden ist, zur entsprechenden Carbonsäure hydrolysiert; oder

h) eine Verbindung der Formel I, worin $R^{10}$ einen Rest der in Anspruch 1 definierten Formel (f) bedeutet, wobei $R^{20}$ Wasserstoff bedeutet, acyliert; oder

i) eine Verbindung der Formel I, worin $R^8$ und $R^9$ zusammen eine zusätzliche Kohlenstoff-Kohlenstoff-Bindung und $R^{10}$ einen Rest der in Anspruch 1 definierten Formel (d) bedeutet, wobei $R^{15}$ Wasserstoff bedeutet, in die entsprechende Verbindung der Formel I, worin $R^8$ und $R^9$ je Wasserstoff, $R^{10}$ einen Rest der in Anspruch 1 definierten Formel (a) und $R^{11}$ Wasserstoff bedeuten, überführt; oder

j) eine Verbindung der Formel I, worin $R^{10}$ einen Rest der in Anspruch 1 definierten Formel (d) bedeutet, in die entsprechende Verbindung der Formel I, worin $R^{10}$ einen Rest der in Anspruch 1 definierten Formel (c) bedeutet, überführt; oder

k) eine saure Verbindung der Formel I mit einer Base bzw. eine basische Verbindung der Formel I mit einer Säure in ein pharmazeutisch verwendbares Salz überführt.

20. Verfahren zur Herstellung von Verbindungen gemäss einem der Ansprüche 13 bis 17, dadurch gekennzeichnet, dass man eine Verbindung der allgemeinen Formel

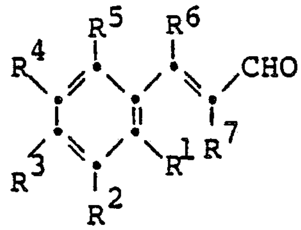

X

worin $R^1$, $R^2$, $R^3$, $R^4$, $R^5$, $R^6$ und $R^7$ die in Anspruch 1 angegebene Bedeutung besitzen,
mit einer Verbindung der allgemeinen Formel

$HC{\equiv}C\text{-}R^{10vi}$ XI

worin $R^{10vi}$ einen Rest der in Anspruch 1 definierten Formel (a), (b), (d) oder (e) oder in Anspruch 19 definierten Formel (f') bedeutet,
umsetzt.

21. Arzneimittel, enthaltend eine Verbindung gemäss einem der Ansprüche 1 bis 17 und ein therapeutisch inertes Excipiens.

22. Arzneimittel gemäss Anspruch 21 mit mucosaprotektiven und/oder magensäuresekretionshemmenden Eigenschaften zur Bekämpfung oder Verhütung von Ulcus ventriculi und/oder duodeni.

23. Verwendung von Verbindungen gemäss einem der Ansprüche 1 bis 17 bei der Bekämpfung oder Verhütung von Krankheiten, insbesondere von Ulcus ventriculi und/oder duodeni, bzw. zur Herstellung von Arzneimitteln gegen Ulcus ventriculi und/oder duodeni.

Patentansprüche für die folgenden Vertragsstaaten: GR, ES

1. Verfahren zur Herstellung von Styrylketonen der allgemeinen Formel

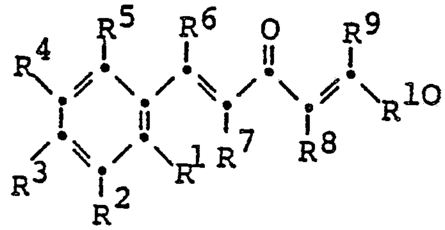

I

worin

$R^1$, $R^2$, $R^3$, $R^4$ und $R^5$ je Wasserstoff, Halogen, niederes Alkyl, niederes Alkoxy-niederes-alkyl, Hydroxy, niederes Alkoxy, niederes Alkenyloxy, niederes Alkinyloxy, niederes Alkoxy-niederes-alkoxy, Acyloxy, Aryl-niederes-alkoxy, niederes Alkylthio, niederes Alkoxy-niederes-alkylthio, niederes Alkenylthio, niederes Alkinylthio, Aryl-niederes-alkylthio, gegebenenfalls substituiertes Amino oder Trifluormethyl oder zwei benachbarte dieser Substituenten gemeinsam und zusammen mit den Kohlenstoffatomen, an welche sie gebunden sind, einen 5-bis 7-gliedrigen Ring bedeuten, wobei von den substituenten $R^1$ bis $R^5$ mindestens zwei Wasserstoff bedeuten und mindestens einer von Wasserstoff verschieden ist;
$R^6$ und $R^7$ je Wasserstoff oder niederes Alkyl bedeuten;
$R^8$ und $R^9$ je Wasserstoff oder niederes Alkyl oder zusammen eine zusätzliche Kohlenstoff-Kohlenstoff-Bindung bedeuten;
$R^{10}$ einen Rest der Formel

$-COOR^{11}$, (a)  $-CONR^{12}R^{13}$, (b)  $-C(R^{14})=O$, (c)

$-C(R^{15})(OR^{16})_2$, (d)  $-C(OR^{17})_3$ (e)  oder

$-C(R^{18})(R^{19})OR^{20}$ (f)  bedeutet;

$R^{11}$ Wasserstoff, niederes Alkyl, niederes Alkenyl, niederes Alkoxy-niederes-alkyl, niederes Alkoxy-niederes-alkoxy-niederes-alkyl, Aryl oder Aryl-niederes-alkyl bedeutet;
$R^{12}$ und $R^{13}$ je Wasserstoff oder niederes Alkyl oder gemeinsam und zusammen mit dem Stickstoffatom einen 5-bis 7-gliedrigen gesättigten heterocyclischen Rest bedeuten;
$R^{14}$ niederes Alkyl, Aryl oder Aryl-niederes-alkyl bedeutet;
$R^{15}$ Wasserstoff, niederes Alkyl, Aryl oder Aryl-niederes-alkyl bedeutet;
$R^{16}$ niederes Alkyl oder niederes Alkoxy-niederes-alkyl bedeutet;
$R^{17}$ niederes Alkyl bedeutet;
$R^{18}$ und $R^{19}$ je Wasserstoff, niederes Alkyl, Aryl oder Aryl-niederes-alkyl bedeuten; und
$R^{20}$ Wasserstoff, niederes Alkyl, niederes Alkoxy-niederes-alkyl, niederes Alkenyl, niederes Alkinyl, Acyl oder Aryl-niederes-alkyl bedeuten;
wobei die im Molekül vorkommende(n) Doppelbindung(en) die E-und/oder Z-Konfiguration aufweist (aufweisen); sowie von pharmazeutisch verwendbaren Salzen von sauren Verbindungen der Formel I mit Basen und von basischen Verbindungen der Formel I mit Säuren, dadurch gekennzeichnet, dass man

a) eine Verbindung der allgemeinen Formel

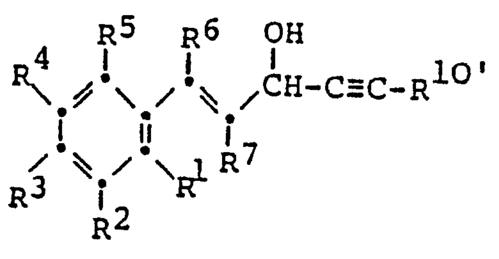

II

worin $R^1$, $R^2$, $R^3$, $R^4$, $R^5$, $R^6$ und $R^7$ obige Bedeutung besitzen und $R^{10'}$ einen Rest der oben definierten Formel (a), (b), (d) oder (e) oder einen Rest der Formel

$$-C(R^{18})(R^{19})OR^{20'}$$

(f')

bedeutet,

worin $R^{18}$ und $R^{19}$ obige Bedeutung besitzen und $R^{20'}$ die oben für $R^{20}$ definierte Bedeutung besitzt, jedoch nicht Wasserstoff bedeutet, wenn $R^{18}$ und/oder $R^{19}$ Wasserstoff bedeutet,
oxydiert; oder

b) eine Verbindung der obigen Formel II, worin $R^{10'}$ einen Rest der oben definierten Formel (a) bedeutet, wobei aber $R^{11}$ nicht Wasserstoff bedeutet, mit einer Base behandelt; oder

c) eine Verbindung der allgemeinen Formel

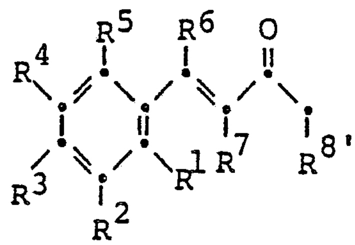

III

worin $R^1$, $R^2$, $R^3$, $R^4$, $R^5$, $R^6$ und $R^7$ obige Bedeutung besitzen und $R^{8'}$ Wasserstoff oder niederes Alkyl bedeutet,
mit einer Verbindung der allgemeinen Formel

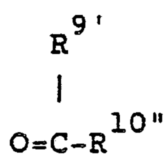

IV

worin $R^{9'}$ Wasserstoff oder niederes Alkyl und $R^{10''}$ einen Rest der oben definierten Formel (a) bedeuten, wobei $R^{11}$ Wasserstoff bedeutet,
umsetzt; oder

d) eine Verbindung der allgemeinen Formel

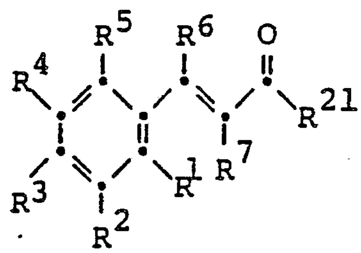

V

worin $R^1$, $R^2$, $R^3$, $R^4$, $R^5$, $R^6$ und $R^7$ obige Bedeutung besitzen und $R^{21}$ eine Abgangsgruppe bedeutet,
mit einer Verbindung der allgemeinen Formel

$H{\equiv}C\text{-}R^{10'''}$ VI

worin $R^{10'''}$ einen Rest der oben definierten Formel (a), (b), (d), (e) oder (f) bedeutet, umsetzt; oder

e) eine Verbindung der allgemeinen Formel

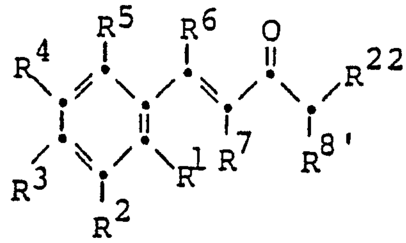

VII

worin $R^1$, $R^2$, $R^3$, $R^4$, $R^5$, $R^6$, $R^7$ und $R^{8'}$ obige Bedeutung besitzen und $R^{22}$ Halogen bedeutet, mit einer Verbindung der allgemeinen Formel

$(R^{23})_3P=CH\text{-}R^{10iv}$ VIII

worin $R^{23}$ einen Arylrest und $R^{10iv}$ einen Rest der oben definierten Formel (a), (b), (d), (e) oder (f) bedeutet, wobei aber $R^{11}$ und $R^{20}$ nicht Wasserstoff bedeuten, umsetzt; oder

f) von einer Verbindung der allgemeinen Formel

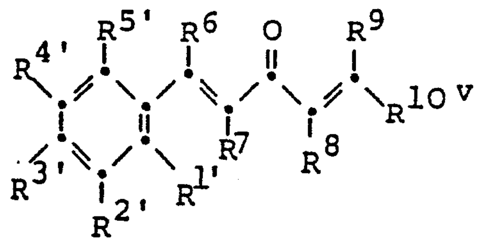

IX

worin $R^{1'}$, $R^{2'}$, $R^{3'}$, $R^{4'}$ und $R^{5'}$ die oben für $R^1$, $R^2$, $R^3$, $R^4$ und $R^5$ angegebene Bedeutung besitzen und maximal drei davon zusätzlich geschütztes Hydroxy, geschütztes Amino oder geschütztes niederes Alkylamino bedeuten können, $R^6$, $R^7$, $R^8$ und $R^9$ obige Bedeutung besitzen, $R^{10v}$ einen Rest der oben definierten Formel (a), (b), (c), (d) oder (e) oder der Formel

$$-C(R^{18})(R^{19})OR^{20''}$$

(f")

worin $R^{18}$ und $R^{19}$ obige Bedeutung besitzen und $R^{20''}$ die für $R^{20}$ angegebene Bedeutung besitzt und zusätzlich eine Schutzgruppe bedeuten kann, wobei das Molekül mindestens eine Schutzgruppe enthält, die Schutzgruppe(n) abspaltet; oder

g) eine Verbindung der Formel I, worin $R^{10}$ einen Rest der oben definierten Formel (a) bedeutet, wobei $R^{11}$ von Wasserstoff verschieden ist, zur entsprechenden Carbonsäure hydrolysiert; oder

h) eine Verbindung der Formel I, worin $R^{10}$ einen Rest der oben definierten Formel (f) bedeutet, wobei $R^{20}$ Wasserstoff bedeutet, acyliert; oder

i) eine Verbindung der Formel I, worin $R^8$ und $R^9$ zusammen eine zusätzliche Kohlenstoff-Kohlenstoff-Bindung und $R^{10}$ einen Rest der oben definierten Formel (d) bedeutet, wobei $R^{15}$ Wasserstoff bedeutet, in die entsprechende Verbindung der Formel I, worin $R^8$ und $R^9$ je Wasserstoff, $R^{10}$ einen Rest der oben definierten Formel (a) und $R^{11}$ Wasserstoff bedeuten, überführt; oder

j) eine Verbindung der Formel I, worin $R^{10}$ einen Rest der oben definierten Formel (d) bedeutet, in die entsprechende Verbindung der Formel I, worin $R^{10}$ einen Rest der oben definierten Formel (c) bedeutet, überführt; oder

k) eine saure Verbindung der Formel I mit einer Base bzw. eine basische Verbindung der Formel I mit einer Säure in ein pharmazeutisch verwendbares Salz überführt.

2. Verfahren gemäss Anspruch 1, dadruch gekennzeichnet, dass man eine Verbindung der Formel I herstellt, worin $R^1$ und $R^2$ je Wasserstoff und $R^3$, $R^4$ und $R^5$ je niederes Alkoxy oder $R^1$ und $R^5$ je Wasserstoff und $R^2$, $R^3$ und $R^4$ je niederes Alkoxy oder $R^1$, $R^2$ und $R^4$ je Wasserstoff und $R^3$ und $R^5$ je niederes Alkoxy oder $R^1$, $R^3$ und $R^4$ je Wasserstoff und $R^2$ und $R^5$ je niederes Alkoxy oder $R^1$, $R^2$ und $R^5$ je Wasserstoff und $R^3$ Hydroxy und $R^4$ niederes Alkoxy oder $R^3$ und $R^4$ zusammen niederes Alkylendioxy oder $R^1$, $R^2$, $R^4$ und $R^5$ je Wasserstoff und $R^3$ Halogen, niederes Alkoxy, niederes Alkylthio oder Trifluormethyl oder $R^1$, $R^2$, $R^3$ und $R^5$ je Wasserstoff und $R^4$ niederes Alkoxy bedeuten.

3. Verfahren gemäss Anspruch 2, dadurch gekennzeichnet, dass man eine Verbindung der Formel I herstellt, worin $R^1$, $R^2$, $R^4$ und $R^5$ je Wasserstoff und $R^3$ Fluor oder Methoxy oder $R^1$, $R^2$, $R^3$ und $R^5$ je Wasserstoff und $R^4$ Methoxy bedeuten.

4. Verfahren gemäss einem der Ansprüche 1 bis 3, dadurch gekennzeichnet, dass man eine Verbindung der Formel I herstellt, worin $R^6$ und $R^7$ je Wasserstoff bedeuten.

5. Verfahren gemäss einem der Ansprüche 1 bis 4, dadurch gekennzeichnet, dass man eine Verbindung der Formel I herstellt, worin $R^8$ und $R^9$ je Wasserstoff oder zusammen eine zusätzliche Kohlenstoff-Kohlenstoff-Bindung bedeuten.

6. Verfahren gemäss einem der Ansprüche 1 bis 5, dadurch gekennzeichnet, dass man eine Verbindung der Formel I herstellt, worin $R^{10}$ einen Rest der in Anspruch 1 definierten Formel (a), (d) oder (f), $R^{11}$ Wasserstoff, niederes Alkyl oder niederes Alkoxy-niederes-alkoxy-niederes-alkyl, $R^{15}$ Wasserstoff, $R^{16}$ niederes Alkyl, $R^{18}$ und $R^{19}$ je Wasserstoff und $R^{20}$ Wasserstoff, niederes Alkoxy-niederes-alkyl oder niederes Alkenyl bedeuten.

7. Verfahren gemäss Anspruch 6, dadurch gekennzeichnet, dass man eine Verbindung der Formel I herstellt, worin $R^{10}$ einen Rest der in Anspruch 1 definierten Formel (a) oder (f), $R^{11}$ Wasserstoff, Methyl oder Methoxyäthoxyäthyl und $R^{20}$ Wasserstoff oder 1-Aethoxyäthyl bedeuten.

8. Verfahren gemäss Anspruch 1, dadurch gekennzeichnet, dass man (E,E)-6-(3-Methoxyphenyl)-4-oxo-2,5-hexadiensäure herstellt.

9. Verfahren gemäss Anspruch 1, dadurch gekennzeichnet, dass man (E,E)-6-(4-Methoxyphenyl)-4-oxo-2,5-hexadiensäure herstellt.

10. Verfahren gemäss Anspruch 1, dadurch gekennzeichnet, dass man (2Z,5E)-6-(4-Methoxyphenyl)-4-oxo-2,5-hexadiensäuremethylester herstellt.

11. Verfahren gemäss Anspruch 1, dadurch gekennzeichnet, dass man (E)-6-(4-Methoxyphenyl)-4-oxo-5-hexen-2-insäure herstellt.

12. Verfahren zur Herstellung von Verbindungen allgemeinen Formel

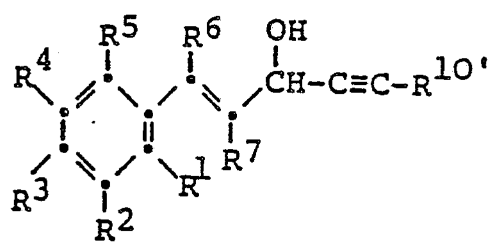

II

worin $R^1$, $R^2$, $R^3$, $R^4$, $R^5$, $R^6$ und $R^7$ die in Anspruch 1 angegebene Bedeutung besitzen und $R^{10'}$ einen Rest der in Anspruch 1 definierten Formel (a), (b), (d) oder (e) oder einen Rest der Formel

$$-C(R^{18})(R^{19})OR^{20'}$$

(f')

bedeutet,

woring $R^{18}$ und $R^{19}$ die in Anspruch 1 angegebene Bedeutung besitzen und $R^{20'}$ die in Anspruch 1 für $R^{20}$ definierte Bedeutung besitzt, jedoch nicht Wasserstoff bedeutet, wenn $R^{18}$ und/oder $R^{19}$ Wasserstoff bedeutet,

dadurch gekennzeichnet, dass man eine Verbindung der allgemeinen Formel

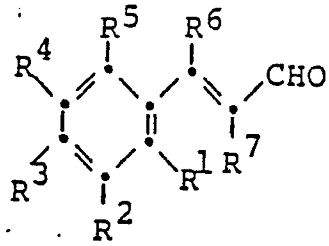

X

worin $R^1$, $R^2$, $R^3$, $R^4$, $R^5$, $R^6$ und $R^7$ die in Anspruch 1 angegebene Bedeutung besitzen, mit einer Verbindung der allgemeinen Formel

$HC \equiv C\text{-}R^{10vi}$ XI

worin $R^{10vi}$ einen Rest der in Anspruch 1 definierten Formel (a), (b), (d) oder (e) oder der oben definierten Formel (f') bedeutet, umsetzt.

13. Verfahren gemäss Anspruch 12, dadurch gekennzeichnet, dass man eine Verbindung der Formel II herstellt, worin $R^{10'}$ einen Rest der in Anspruch 1 definierten Formel (a) bedeutet.

14. Verfahren gemäss Anspruch 13, dadurch gekennzeichnet, dass man eine Verbindung der Formel II herstellt, worin $R^{10'}$ einen Rest der in Anspruch 1 definierten Formel (a) und $R^{11}$ niederes Alkyl oder niederes Alkoxy-niederes-alkoxy-niederes-alkyl bedeuten.

15. Verfahren gemäss Anspruch 12, dadurch gekennzeichnet, dass man (E)-4-Hydroxy-6-(4-methoxyphenyl)-5-hexen-2-insäure-methylester herstellt.

16. Verfahren gemäss Anspruch 12, dadurch gekennzeichnet, dass man (E)-4-Hydroxy-6-(4-methoxyphenyl)-5-hexen-2-insäure-[2-(2-methoxyäthoxy)äthyl]ester herstellt.

17. Verfahren zur Herstellung von Arzneimitteln, insbesondere zur Verwendung bei der Bekämpfung oder Verhütung von Ulcus ventriculi und/oder duodeni, dadurch gekennzeichnet, dass man eine oder mehrere der Verbindungen der in Anspruch 1 bzw. 12 definierten Formeln I oder II oder der pharmazeutisch verwendbaren Salze der Verbindungen der Formel I und erwünschtenfalls einen oder mehrere andere therapeutische Wirkstoffe zusammen mit einem oder mehreren therapeutisch inerten Excipientien in eine galenische Darreichungsform bringt.

18. Verwendung von Verbindungen der in Anspruch 1 bzw. 12 definierten Formeln I und II und von pharmazeutisch verwendbaren Salzen von Verbindungen der Formel I zur Herstellung von Arzneimitteln gegen Ulcus ventriculi und/oder duodeni.